# EUROPEAN PATENT APPLICATION

(11) **EP 4 480 945 A1**
(43) Date of publication of application: **25.12.2024**
(21) Application number: 23756375.4
(22) Date of filing: 15.02.2023
(51) Int. Cl.: C07C 319/22, C07B 61/00, C07C 321/14, C10M 135/04

(54) **METHOD FOR PRODUCING OLEFIN SULFIDE**

(30) Priority: 18.02.2022 JP 2022024040; 30.09.2022 JP 2022157912
(71) Applicant: DIC Corporation, Tokyo 174-8520 (JP); Kyushu University, National University Corporation, Nishi-ku Fukuoka-shi Fukuoka 819-0395 (JP)
(72) Inventor: MATSUEDA Hironobu, Kamisu-shi, Ibaraki 314-0193 (JP); SAKATA Hiroshi, Kamisu-shi, Ibaraki 314-0193 (JP); OTSUKI Shujiro, Kamisu-shi, Ibaraki 314-0193 (JP); YAMAMOTO Eiji, Fukuoka-shi, Fukuoka 819-0395 (JP); TOKUNAGA Makoto, Fukuoka-shi, Fukuoka 819-0395 (JP); MURAYAMA Haruno, Fukuoka-shi, Fukuoka 819-0395 (JP); TAKAKI Yuta, Fukuoka-shi, Fukuoka 819-0395 (JP); KAWAI Yasutaka, Fukuoka-shi, Fukuoka 819-0395 (JP); TAKAKURA Kei, Fukuoka-shi, Fukuoka 819-0395 (JP); KIMURA Moemi, Fukuoka-shi, Fukuoka 819-0395 (JP)
(74) Representative: Kraus & Lederer PartGmbB
(86) International application number: PCT/JP2023/005088
(87) International publication number: WO 2023/157850

(57) **Abstract**

Provided is a method for producing sulfurized olefin, the method including reacting an olefin compound (a) represented by

R¹R²C=CHR³ ··· (1)

(in Formula (1), R¹ and R² each are alkyl groups, respectively, R³ is a hydrogen atom or an alkyl group, and the total number of carbon atoms of R¹, R² and R³ is 2 to 20) with sulfur in the presence of hydrogen to give a dialkyl polysulfide (A).

## Description

### Technical Field

The present invention relates to a method for producing sulfurized olefin.

The present application claims priority based on Japanese Patent Application No. 2022-024040 filed in Japan on February 18, 2022, and Japanese Patent Application No. 2022-157912 filed in Japan on September 30, 2022, the contents of which are incorporated herein by reference.

### Background Art

Lubricant oils are used during processing of metal parts represented by automotive applications. Sulfur-based compounds are widely used as lubricant oil additives because the addition of the sulfur-based compounds to lubricant oils improves processability by reducing friction generated between tools and metal parts during processing. Examples of the sulfur-based lubricant oil additives mainly include dark-colored sulfurized oils, light-colored sulfurized oils, and light-colored sulfurized olefins. In recent years, however, the light-colored sulfurized oils and the light-colored sulfurized olefins having relatively low odor have been desired in consideration of processing environments and other factors.

Conventionally, as a method for producing the dark-colored lubricant oil additives, a method in which olefin and sulfur are subjected to a high-temperature radical reaction in the presence of basic catalysts such as amines. Examples of a methods for producing the light-colored lubricant oil additives include (1) a method for subjecting olefins, sulfur, and hydrogen sulfide to an addition reaction in the presence of the basic catalysts such as amines and (2) a method for subjecting mercaptan, sulfur, and others compounds to the addition reaction in the presence of the basic catalysts such as inorganic/organic bases or alkoxylated compounds.

As the production method (1) described above, for example, a method for producing dialkyl polysulfide including a first step of reacting an olefin compound (a) represented by

R¹R²C=CHR³ ··· (1)

(in the formula, R¹ and R² each are alkyl groups; R³ is a hydrogen atom or an alkyl group, and the total number of carbon atoms of R¹, R², and R³ is 2 to 20) with sulfur in the presence of hydrogen sulfide to give crude dialkyl polysulfide (A), and a second step of reacting the crude dialkyl polysulfide (A) with sulfide of an alkali metal in a solvent including alcohol to reduce the number of sulfur atoms in the crude dialkyl polysulfide (A) has been disclosed (PTL 1).

As the production method (2) described above, for example, a method for producing organopolysulfide compounds in which mercaptan and elemental sulfur are contacted under conditions sufficient to generate a reaction medium including crude organopolysulfide in the presence of sodium hydroxide and ethoxylated alcohol, and thereafter the reaction medium is brought into contact with an acid has been disclosed (PTL 2).

### Citation List

### Patent Literature

PTL 1: WO 2015/046008
PTL 2: Japanese Unexamined Patent Application Publication No. H08-231496

### Summary of Invention

### Technical Problem

In the production method described in Patent Literature 1, the olefin compound is reacted with sulfur in the presence of hydrogen sulfide to give a dialkyl polysulfide, and thus hydrogen sulfide is required for the production of the sulfurized olefin. However, the high-pressure hydrogen sulfide gas required for the above reaction is difficult to obtain and difficult to handle. Only a few companies in the world can currently produce light-colored polysulfide products. Accompanying with the limited production sites for the hydrogen sulfide gas, the production sites for polysulfide products are also limited. Furthermore, although sodium hydroxide or potassium hydroxide is used as a basic catalyst, the use of metal catalysts and zeolites is not disclosed.

In the production method described in Patent Literature 2, the organic polysulfide compounds are obtained by reacting mercaptans such as thiols with sulfur in the presence of sodium hydroxide and ethoxylated alcohols. However, hydrogen sulfide is used in the production of thiol raw materials, and thus hydrogen sulfide is eventually required for the production of the sulfurized olefins. In addition, although the mercaptan raw materials are required for the production of the sulfurized olefin, the mercaptan raw materials are expensive and the types of available mercaptan raw materials are limited. Consequently, the obtained polysulfide structures are limited.

An object of the present invention is to provide a method for producing sulfurized olefin in which sulfurized olefin having light color and less odor can be easily produced at low cost without using hydrogen sulfide. Solution to Problem

As a result of intensive study for achieving the above object, the inventors of the present invention have found that dialkyl polysulfides having various polysulfide structures with light color and less odor are easily obtained by reacting an olefin compound having two alkyl groups bonded to the carbon atom at the 2-position of 1-olefin with sulfur in the presence of hydrogen. In particular, the inventors of the present invention have found that the use of hydrogen (gas), which is becoming more readily available as a next-generation fuel, as a raw material allows the light-colored dialkyl polysulfides to be produced without using difficult-to-obtain high-pressure hydrogen sulfide gas or expensive mercaptans as raw materials. The inventors of the present invention have also found that the yield of the dialkyl polysulfides can be improved by using metal elements as catalysts or metal elements and zeolites as catalysts in the above reaction.

Therefore, the present invention provides the following means.
[1] A method for producing sulfurized olefin, the method including
   reacting an olefin compound (a) represented by

   R¹R²C=CHR³ ... (1)

   (in Formula (1), R¹ and R² each are alkyl groups; R³ is a hydrogen atom or an alkyl group, and the total number of carbon atoms of R¹, R², and R³ is 2 to 20) with sulfur in the presence of hydrogen to give dialkyl polysulfide (A).
[2] The method for producing sulfurized olefin according to [1], in which a pressure of supplied hydrogen in reacting the olefin compound (a) with sulfur is 0.1 MPa or more and 10 MPa or less.
[3] The method for producing sulfurized olefin according to [1] or [2], in which a heating temperature in reacting the olefin compound (a) with sulfur in the presence of hydrogen is 100°C or more and 200°C or less.
[4] The method for producing sulfurized olefin according to any one of [1] to [3], in which the olefin compound (a) is reacted with sulfur in the presence of hydrogen and a metal element.
[5] The method for producing sulfurized olefin according to [4], in which the metal element is one or more metal elements selected from Group 6 to Group 11.
[6] The method for producing sulfurized olefin according to [4] or [5], in which the metal element is a metal element constituting a metal oxide or a metal sulfide.
[7] The method for producing sulfurized olefin according to any one of [4] to [6], in which an added amount of the metal element relative to the olefin compound (a) is 0.1 mol% or more and 10 mol% or less.
[8] The method for producing sulfurized olefin according to any one of [4] to [7], in which the olefin compound (a) is reacted with sulfur in the presence of hydrogen, the metal element, and zeolite.
[9] The method for producing sulfurized olefin according to [8], in which the zeolite has basicity.
[10] The method for producing sulfurized olefin according to [8] or [9], in which the zeolite has an X-type or A-type pore structure.
[11] The method for producing sulfurized olefin according to any one of [8] to [10], in which an added amount of the zeolite is 2.0 parts by mass or more and 25 parts by mass or less relative to 100 parts by mass of the olefin compound (a).
[12] The method for producing sulfurized olefin according to [1], in which a total number of carbon atoms of R¹ and R² in the general formula (1) is 2 to 14 and R³ is a hydrogen atom.
[13] The method for producing sulfurized olefin according to [1], in which the olefin compound (a) is diisobutylene.

### Advantageous Effects of Invention

According to the present invention, a method for producing sulfurized olefin in which sulfurized olefin having light color and less odor can be easily produced at low cost without using hydrogen sulfide can be provided.

### Brief Description of Drawings

FIG. 1 is a schematic view illustrating a method for producing dialkyl polysulfide (A) in Example 1.
FIG. 2 is a chart illustrating the dialkyl polysulfide (A) obtained in Example 1 and peaks corresponding to the dialkyl polysulfides of each n value (n = 3 to 8).
FIG. 3 is a chart illustrating the dialkyl polysulfide (A) obtained in Example 41 and peaks corresponding to the dialkyl polysulfides of each n value (n = 3 to 8).
FIG. 4 is a chart illustrating the dialkyl polysulfide (A) obtained in Example 42 and peaks corresponding to the dialkyl polysulfides of each n value (n = 3 to 8).
FIG. 5 is a graph illustrating a relation between a reaction time and a conversion ratio in the case where a hydrogen pressure is set to 5.0 MPa in Examples.
FIG. 6 is a graph illustrating a relation between the reaction time and a total yield in the case where the hydrogen pressure is set to 5.0 MPa in Examples.
FIG. 7 is a graph illustrating a relation between the reaction time and the conversion ratio in the case where the hydrogen pressure is set to 3.0 MPa in Examples.
FIG. 8 is a graph illustrating a relation between the reaction time and the total yield in the case where the hydrogen pressure is set to 3.0 MPa in Examples. Description of Embodiments

The method for producing sulfurized olefin according to the present invention is a method for producing sulfurized olefin, the method including reacting an olefin compound (a) represented

by R¹R²C=CHR³ ··· (1)

(in the formula, R¹ and R² each are alkyl groups; R³ is a hydrogen atom or an alkyl group, and the total number of carbon atoms of R¹, R², and R³ is 2 to 20) with sulfur in the presence of hydrogen to give dialkyl polysulfide (A).

### (Olefin Compound (a))

The olefin compound (a) used in the above step has the structure represented by the general formula (1) as described above. From the viewpoint that the compound is easily available and can be preferably used as an extreme pressure additive due to excellent solubility of the dialkyl polysulfide serving as the final product into base oil, among the olefin compound (a), an olefin compound having a total number of carbon atoms in R¹ and R² of 2 to 18 is preferable and an olefin compound having a total number of carbon atoms in R¹ and R² of 2 to 14 is more preferable.

In addition, from the viewpoint that the compound is easily available and can be preferably used as the extreme pressure additive due to excellent solubility of the dialkyl polysulfide (A) serving as the final product into a base oil, among the olefin compound (a), an olefin compound having a hydrogen atom as R³ is preferable.

Therefore, among the olefin compounds (a) having the structure represented by the general formula (1), an olefin compound having a total number of carbon atoms in R¹ and R² of 2 to 18 and a hydrogen atom as R³ is preferable, and an olefin compound having a total number of carbon atoms of R¹ and R² of 2 to 14 and a hydrogen atom as R³ is more preferable.

Examples of the olefin compound (a) used in the present embodiment include diisobutylene, isobutylene, 3-methylpentene, 4-methylheptene, 5-methylundecene, and 3,6-dimethylhexanedecene. Of these compounds, diisobutylene is preferable because this compound provides dialkyl polysulfide having a high sulfur content and less odor.

In the production method according to the present embodiment, other olefin compounds other than the olefin compound (a) can be used together with the olefin compound (a) to the extent that the effect of the present invention is not impaired. Examples of such olefin compounds include 1-butene, 1-pentene, 1-hexene, 1-octene, 1-decene, 1-dodecene, 1-tetradecene, 1-hexadecene, 1-octadecene, 1-eicosene, or mixtures thereof.

### (Sulfur)

Sulfur used in the present embodiment is not particularly limited, and may be in a solid state, for example, in the form of small lumps, flakes, or powders, or in a melted state (liquid). Of these states, sulfur in the melted state is particularly preferable from the viewpoint of ease of a charging operation in large scale production.

In the above step, from the viewpoint of solubility, a ratio of the olefin compound (a) and sulfur to be used is preferably 1.0 mol to 5.0 mol per 1 mol of the olefin (a) in terms of S element (1.0 molar equivalent to 5.0 molar equivalents in terms of S equivalent and 0.125 molar equivalent to 0.625 molar equivalent in terms of S₈), more preferably 1.0 mol to 4.0 mol per 1 mol of the olefin (a) in terms of S element (1.0 molar equivalent to 4.0 molar equivalents in terms of S equivalent and 0.125 molar equivalent to 0.5 molar equivalent in terms of S₈), and further preferably 1.5 mol to 3.0 mol per 1 mol of the olefin (a) in terms of S element (1.5 molar equivalents to 3.0 molar equivalents in terms of S equivalent and 0.1875 molar equivalent to 0.375 molar equivalent in terms of S₈).

### (Hydrogen)

Hydrogen used in the present embodiment is typically hydrogen gas. Hydrogen gas is more widely distributed and easily obtained as compared to hydrogen sulfide. In addition, although flammable, hydrogen gas is non-toxic and odorless, and thus the handling is easy as compared to the handling of hydrogen sulfide. Hydrogen gas is not limited, and may be, for example, 99.99% or more, 99.999% or more, or 99.9999% or more.

A method for obtaining hydrogen gas is not particularly limited. Hydrogen gas distributed in the market may be obtained or hydrogen gas may be produced using a reforming method, in which hydrogen is obtained by reacting hydrocarbons such as methane with water vapor, or the electrolysis method, in which hydrogen is obtained by electrolyzing water.

When the olefin compound (a) is reacted with sulfur in the presence of hydrogen, the pressure of supplied hydrogen is preferably 0.1 MPa or more and 10.0 MPa or less, more preferably 1.0 MPa or more and 6.0 MPa or less, and further preferably 1.0 MPa or more and 4.0 MPa or less. Setting the pressure of the supplied hydrogen to 0.1 MPa or more and 10.0 MPa or less allows the yield of the obtained dialkyl polysulfide (A) to be improved.

A heating temperature in reacting the olefin compound (a) with sulfur in the presence of hydrogen is preferably 100°C or more and 200°C or less, preferably 100°C or more and 160°C or less, further preferably 100°C or more and 140°C or less, and particularly preferably 120°C or more and 140°C or less. Setting the heating temperature during the reaction to 100°C or more and 200°C or less allows the yield of the obtained dialkyl polysulfide (A) to be improved while the productivity is being improved by accelerating the reaction time.

In the above step, the olefin compound (a) is reacted with sulfur in the presence of hydrogen to give the dialkyl polysulfide (A). The step is not limited to this step and the olefin compound (a) is reacted with sulfur in the presence of hydrogen and a metal element. Subjecting the olefin compound (a) to the contact reaction with sulfur using metal element as a catalyst in the presence of hydrogen allows the yield of the dialkyl polysulfide (A) to be improved.

### (Metal Elements)

The metal element is preferably one or more metal elements selected from Group 6 to Group 11. Examples of such a metal element include the followings.
Group 6: Chromium (Cr), Molybdenum (Mo), Tungsten (W)
Group 7: Manganese (Mn), Technetium (Tc), Rhenium (Re)
Group 8: Iron (Fe), Ruthenium (Ru), Osmium (Os)
Group 9: Cobalt (Co), Rhodium (Rh), Iridium (Ir)
Group 10: Nickel (Ni), Palladium (Pd), Platinum (Pt)
Group 11: Copper (Cu), Silver (Ag), Gold (Au)

The metal element is preferably a metal element that constitutes, for example, metal oxides or metal sulfides. In the case where the metal element is, for example, a metal element constituting a metal oxide, the olefin compound (a) is reacted with sulfur in the presence of hydrogen and the metal oxide. In the case where the metal element is a metal element constituting a metal sulfide, the olefin compound (a) is reacted with sulfur in the presence of hydrogen and the metal sulfide. The valence of the metal element constituting the metal oxide or the metal sulfide is not particularly limited and can take various values such as +1 and +2.

In the case where the metal element is a metal element constituting metal oxides, examples of the metal oxides include, but are not limited to the following compounds.
Metal oxides of Group 6: CrO₃, MoO₃, WO₃
Metal oxides of Group 7: Mn₂O₃, Tc₂O₇, ReO₃
Metal oxides of Group 8: Fe₂O₃, RuO₂, OsO₄
Metal oxides of Group 9: CoO, Rh₂O₃, IrO₂
Metal oxides of Group 10: NiO, PdO, PtO₂
Metal oxides of Group 11: CuO, Ag₂O, Au₂O₃

In the case where the metal element is a metal element constituting metal sulfides, the metal sulfides include, but are not limited to the following compounds.
Metal sulfides of Group 6: Cr₂S₃, MoS₂, WS₂
Metal sulfides of Group 7: MnS, ReS₂
Metal sulfides of Group 8: FeS, RuS₂, OsS₂
Metal sulfides of Group 9: CoS, Rh₂S₃, IrS₂
Metal sulfides of Group 10: Ni₃S₂, PdS, PtS
Metal sulfides of Group 11: CuS, Ag₂S, Au₂S

In the case where the metal element is, for example, cobalt (Co), cobalt oxide can be used as the metal oxide and cobalt sulfide as the metal sulfide. In the case where cobalt oxide is used as the metal oxide, the olefin compound (a) is reacted with sulfur in the presence of hydrogen and the cobalt oxide. The cobalt oxide to be used is not particularly limited. Examples of the cobalt oxide include CoO, Co₂O₃, and Co₃O₄. In the case where cobalt sulfide is used as the metal sulfide, the olefin compound (a) is reacted with sulfur in the presence of hydrogen and the cobalt sulfide. The cobalt sulfide to be used is not particularly limited. Examples of the cobalt sulfide include CoS, Co₉S₈, CoS₂, and Co₃S₄. Of these compounds, cobalt oxide, in particular Co₃O₄ is preferable from the viewpoint of improving the yield of the dialkyl polysulfide (A) .

The added amount of the metal element relative to the olefin compound (a) is preferably 0.1 mol% or more and 10 mol% or less, more preferably 1.0 mol% or more and 10 mol% or less, and further preferably 2.0 mol% or more and 7.0 mol% or less. The added amount of the metal element relative to the olefin compound (a) of 0.1 mol% or more and 10 mol% or less allows the reaction between the olefin compound (a) and sulfur in the presence of hydrogen to be promoted and the yield of the obtained dialkyl polysulfide (A) to be improved.

### (Zeolite)

In the above step, the olefin compound (a) is reacted with sulfur in the presence of hydrogen to give the dialkyl polysulfide (A). The step is not limited to this step, and the olefin compound (a) is reacted with sulfur in the presence of hydrogen, the metal element, and zeolite. Using the metal element and zeolite as catalysts and subjecting the olefin compound (a) to the contact reaction with sulfur using the two catalysts in the presence of hydrogen allow the yield of the dialkyl polysulfide (A) to be improved.

Zeolite is crystalline aluminosilicate, which has a backbone structure of regularly linked silica and alumina, and also contains cations as ion exchange sites in the pore structure. The pore structure defined by the zeolite backbone structure is not particularly limited. Examples of the structure include LTA (type A), FER (ferrierite), MWW (MCM-22), MFI (ZSM-5), MOR (mordenite), LTL (type L), FAU (type Y and type X), and BEA (type beta). Of these types, zeolite having the X-type or A-type pore structure is preferable from the viewpoint of easy availability and economic efficiency.

The zeolite preferably has basicity. Examples of the zeolite having basicity include zeolite containing alkali metal or alkaline earth metal cations. Examples of the alkali metal include sodium (Na) and potassium (K), and examples of the alkaline earth metal include magnesium (Mg) and calcium (Ca).

From the viewpoint of improving the yield of the dialkyl polysulfide (A), the zeolite is preferably the Na-X type zeolite, which has an X-type backbone structure and contains sodium ions, or the Na-A type zeolite, which has an A-type backbone structure and contains sodium ions. Of these zeolites, the Na-X type zeolite is more preferable.

The added amount of zeolite is preferably 2.0 parts by mass or more and 25 parts by mass or less, more preferably 2.0 parts by mass or more and 15 parts by mass or less, further preferably 3.0 parts by mass or more and 10 parts by mass or less, and particularly preferably 4.0 parts by mass or more and 8.0 parts by mass or less relative to 100 parts by mass of the olefin compound (a). The added amount of zeolite of 2.0 parts by mass or more and 25 parts by mass or less relative to 100 parts by mass of the olefin compound (a) allows the yield of the obtained dialkyl polysulfide (A) to be further improved.

The dialkyl polysulfide (A) obtained by the production method according to the present embodiment is a mixture of dialkyl polysulfides having different numbers of sulfur atoms. The content (yield) of each of the dialkyl polysulfides having different numbers of sulfur atoms can be determined by the peak areas of the chart obtained by ultra high-performance liquid chromatography (hereinafter also referred to as "UPLC") measurement. The total yield of the dialkyl polysulfides (A) can be determined from the total of each yield of the dialkyl polysulfides having different numbers of sulfur atoms.

In the case of applications in which sulfurization of passive coating films for difficult-to-process metals or other materials is desired to be promoted, the amount of active sulfur in the dialkyl polysulfide (A) obtained by the production method according to the present embodiment is 20% by mass to 50% by mass and more preferably 30% by mass to 45% by mass relative to the total sulfur content as the standard. From the viewpoint of effectively forming a metal sulfide film on a metal surface and being less likely to cause the corrosion of the metal surface, the amount of the active sulfur is preferably 0.1% by mass to 30% by mass and more preferably 0.5% by mass to 20% by mass relative to the total sulfur content as the standard. The amount of the active sulfur in the present invention is determined by the method specified in ASTM-D1662.

The dialkyl polysulfide (A) (sulfurized olefin) obtained by the production method according to the present embodiment is represented by the following general formula (2) .

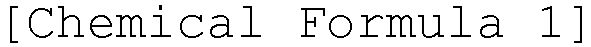

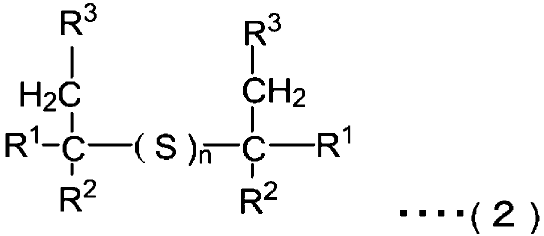

(In the formula, R¹ and R² each are alkyl groups; R³ is a hydrogen atom or an alkyl group; the total number of carbon atoms of one R¹, one R², and one R³ is 2 to 20; and n is an integer of 1 to 8.)

In the general formula (2), from the viewpoint that the dialkyl polysulfide (A) has excellent solubility in a base oil and can be preferably used as an extreme pressure additive, the total number of carbon atoms of R¹ and R² is preferably 2 to 18, and the total number of carbon atoms of R¹ and R² is more preferably 2 to 14.

In the general formula (2), from the viewpoint that the dialkyl polysulfide (A) has excellent solubility in a base oil and can be preferably used as an extreme pressure additive, R³ is preferably a hydrogen atom.

Therefore, in the general formula (2), it is preferable that the total number of carbon atoms of R¹ and R² be 2 to 18 and R³ be a hydrogen atom and it is more preferable that the total number of carbon atoms of R¹ and R² be 2 to 14 and R³ be a hydrogen atom.

From the viewpoint that the dialkyl polysulfide (A) effectively forms a metal sulfide film on a metal surface, is less likely to cause corrosion of the metal surface, and can be suitably used as an extreme pressure additive, n is preferably an integer of 2 to 8 and more preferably an integer of 3 to 8.

In the method for producing sulfurized olefin according to the present embodiment, the dialkyl polysulfide (A) may be obtained by reacting the olefin compound (a) represented by

R¹R²C=CHR³ ··· (1)

(in the formula, R¹ and R² each are an alkyl group; R³ is a hydrogen atom or an alkyl group; and the total number of carbon atoms of R¹, R², and R³ is 2 to 20) with sulfur in the presence of hydrogen and a radical scavenger. The radical scavenger is not particularly limited as long as the radical scavenger has radical scavenging ability. Examples of the radical scavenger include 2,6-di-tert-butyl-p-cresol. The reaction between the olefin compound (a) represented by the general formula (1) and sulfur proceeds even in the presence of the radical scavenger, and thus it is assumed that the reaction is not a radical reaction because the influence of the radical scavenger is small. Therefore, the dialkyl polysulfide (A) can also be obtained in this production method.

The production method according to the present embodiment allows the metal sulfide film to be effectively formed on the metal surface and the dialkyl polysulfide (A), which can be suitably used as an extreme pressure additive, to be efficiently obtained. The obtained dialkyl polysulfide (A) effectively forms the metal sulfide film on the metal surface and can be suitably used as the extreme pressure additive.

The extreme pressure additive may be formed of the dialkyl polysulfide (A) alone or may include dialkyl polysulfide (B) other than the dialkyl polysulfide (A). The dialkyl polysulfide (B) is represented, for example, by the following general formula (3). 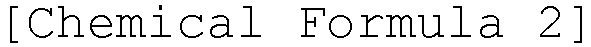 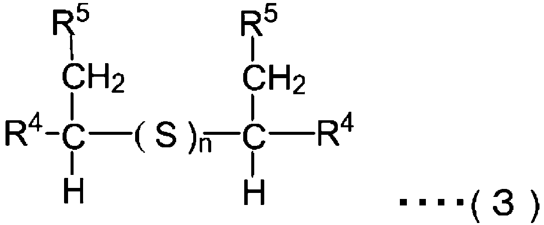 (in the formula, R⁴ and R⁵ each are a hydrogen atom or an alkyl group; the total number of carbon atoms of one R⁴ and one R⁵ is 4 to 20; and n is an integer of 1 to 6).

In the general formula (3), examples of R⁴ and R⁵ include linear alkyl groups and branched alkyl groups. Examples of the linear alkyl groups include a methyl group, an ethyl group, a n-butyl group, a n-pentyl group, a n-octyl group, a n-decyl group, a n-dodecyl group, a n-hexadecyl group, and a n-octadecyl group. Examples of the branched alkyl groups include a 3-methylpentyl group, a 4-methylheptyl group, a 5-methylundecyl group, and a 3,6-dimethylhexanedecyl group.

Of the dialkyl polysulfides (B), the total number of carbon atoms of one R⁴ and one R⁵ is preferably 4 to 16 and more preferably 4 to 10 from the viewpoint that a high sulfur content can be maintained and the excellent metal sulfide film can be formed on the metal surface.

In the case of applications in which sulfurization of passive coating films for difficult-to-process metals or other materials is desired to be promoted, the amount of the active sulfur in the dialkyl polysulfide (B) is preferably 20% by mass to 50% by mass and more preferably 30% by mass to 45% by mass relative to the total sulfur content as the standard. From the viewpoint of effectively forming the metal sulfide film on the metal surface and being less likely to cause corrosion of the metal surface, the amount of the active sulfur is preferably 0.1% by mass to 30% by mass and more preferably 0.5% by mass to 20% by mass relative to the total sulfur content as the standard. The amount of the active sulfur in the dialkyl polysulfide (B) is determined by the method specified in ASTM-D1662.

The dialkyl polysulfide (B) is obtained by reacting an olefin compound (b) represented by

R⁴HC=CHR⁵ ··· (4)

(in the formula, R⁴ and R⁵ each are a hydrogen atom or an alkyl group, and the total number of carbon atoms of R⁴ and R⁵ is 4 to 20) with sulfur in the presence of hydrogen.

The olefin compound (b) used in the production method has the structure represented by the general formula (4), as described above. Of the dialkyl polysulfides (B), an olefin compound having the total number of carbon atoms of R⁴ and R⁵ of 4 to 16 is preferably used and an olefin compound having the total number of carbon atoms of R⁴ and R⁵ of 4 to 10 is more preferably used as the olefin compound (b) from the viewpoint that a high sulfur content can be maintained and the excellent metal sulfide film can be formed on the metal surface. In addition, an olefin compound having an alkyl group having a number of carbon atoms of 4 to 16 as R⁴ and a hydrogen atom as R⁵ is preferably used and an olefin compound having an alkyl group having a number of carbon atoms of 4 to 10 as R⁴ and a hydrogen atom as R⁵ is more preferably used as the olefin compound (b) from the viewpoint that the reactivity is excellent and the dialkyl polysulfide (B) is efficiently obtained.

Examples of the olefin compound (b) include linear 1-olefin and branched 1-olefin having a branched structure other than the ends. Examples of the linear 1-olefin include 1-butene, 1-pentene, 1-hexene, 1-octene, 1-decene, 1-dodecene, 1-tetradecene, 1-hexadecene, 1-octadecene, 1-eicosene, or mixtures thereof.

Of the olefin compounds (b), the linear 1-olefin is preferable because the linear 1-olefin is industrially readily available and reaction of the linear 1-olefin with sulfur proceeds easily. Of the linear 1-olefins, olefin compounds having a number of carbon atoms of 6 to 18 are preferable because these olefin compounds have a low flow point and thus can maintain a liquid state at room temperature. Of these olefin compounds, olefin compounds having a number of carbon atoms of 6 to 12 are more preferable.

As sulfur and hydrogen, the same sulfur and hydrogen used in the production method described above may be used.

### (Lubricating Fluid Composition)

A lubricating fluid composition can include the extreme pressure additive obtained in the present embodiment and a base oil. The base oil is not particularly limited and can be used by appropriately selecting from mineral oils, synthetic oils, and other oils depending on the purpose of use, conditions of use, and other factors. Examples of the mineral oils include distillate oils obtained by atmospheric distillation of paraffinic oils, intermediate oils, and naphthenic oils or by reduced pressure distillation of residues after atmospheric distillation, or refined oils obtained by refining these oils by solvent refining, hydrogenated refining, wax removal treatment, white earth treatment, or other refining processes. Examples of the synthetic oils include low molecular weight polybutene, low molecular weight polypropylene, α-olefin oligomers having a number of carbon atoms of 8 to 14 and hydrogenated products thereof, polyol esters such as fatty acid esters of trimethylolpropane and fatty acid esters pentaerythritol, ester-based compounds such as dibasic acid esters, aromatic polycarboxylic acid esters, and phosphate esters, alkyl aromatic compounds such as alkyl benzenes and alkyl naphthalenes, polyglycol oils such as polyalkylene glycols, and silicone oils. These oils may be appropriately used alone or in combination of two or more of the oils.

The blend ratio of the extreme pressure additive to the base oil in the lubricating fluid composition is not particularly limited. Usually, the blend ration is 0.01 part by mass to 50 parts by mass and preferably 0.05 part by mass to 20 parts by mass per 100 parts by mass in terms of dialkyl polysulfide in the extreme pressure additive relative to the base oil.

The lubricating fluid composition can also be used as grease by further adding a thickener to the lubricating fluid composition. Examples of the thickener that can be used here include soap-based products such as metallic soap-based products and composite soap-based products, or urea-based products. In the case where these thickeners are used, the thickeners may be previously mixed in the base oil to homogenize the mixture.

The lubricating fluid composition may include additives in addition to the extreme pressure additive and the base oil. For example, additives such as oiliness agents, wear-resistant agents, extreme pressure agents, other rust inhibitors, corrosion inhibitors, antifoam agents, cleaning dispersants, flow point depressants, viscosity index improvers, antioxidants, emulsifiers, antiemulsifiers, mold inhibitors, friction modifiers, and surfactants can be used together depending on the target application and performance.

Specific examples of various types of the additives may be exemplified as follows. Examples of the oiliness agents include long-chain fatty acids (oleic acid); examples of the wear-resistant agents include phosphate esters and metal dithiophosphate salts; examples of the extreme pressure agents include organosulfur compounds and organohalogen compounds; examples of other rust inhibitors include carboxylic acids, amines, alcohols, and esters; examples of the corrosion inhibitors include nitrogen compounds (such as benzotriazole) and compounds containing sulfur and nitrogen (1,3,4-thiadiazolyl-2,5-bis-dialkyl dithiocarbamate); examples of the antifoam agents include silicone oils, metal soaps, fatty acid esters, and phosphate esters; examples of the cleaning dispersants include neutral and basic sulfonate and phenates (metal salt type), succinimide, esters, and benzylamine copolymer polymers; examples of the flow point depressants include condensation products of chlorinated paraffins with naphthalene or phenol, polyalkylacrylates and methacrylates, polybutene, polyalkylstyrene, and polyvinyl acetate; examples of the viscosity index improvers include polymethacrylates, polyisobutylene, olefin copolymers, and polyalkylstyrene; examples of the antioxidants include amines, hindered phenols, zinc thiophosphate, and trialkyl phenols; examples of the emulsifiers include esters of sulfuric acid, sulfonic acid, and phosphoric acid, fatty acid derivatives, amine derivatives, quaternary ammonium salts, and polyoxyethylene-based activators; examples of the antiemulsifiers include quaternary ammonium salts, sulfated oils, and phosphate esters; and examples of the mold inhibitors include phenolic compounds, formaldehyde donor compounds, and salicylanilide-based compounds.

The lubricating fluid composition is a composition in which the extreme pressure additive, the base oil, and if necessary, the thickener and other additives are homogeneously blended. The blending method is not particularly limited. At this time, the mixture can be heated to 30°C to 60°C for homogenization.

The applications of the lubricating fluid composition are not particularly limited. For example, the lubricating fluid composition can be used as the lubricant composition and can be used as lubricant oils for automobiles used in internal combustion engines, automatic transmissions, buffers, drive system components such as power steering, and gears; metalworking oils used in cutting processes, grinding processes, plastic forming processes, and other metalworking processes; and hydraulic oils serving as power transmission fluids used for operations in power transmission, power control and buffers for hydraulic systems such as hydraulic equipment and devices. In particular, the lubricating fluid composition according to the present invention can reduce the degree of swelling to the sealant (chloroprene rubber, nitrile rubber, and the like) of a gearbox used when the lubricating fluid composition is used as a gear oil as compared to conventional products. Consequently, the lubricating fluid composition can be suitably used for applications in which the lubricating fluid composition is brought into contact with the sealant.

### [Examples]

Hereinafter, Examples of the present invention will be described. The present invention, however, is not limited to Examples described below.

### (Example 1)

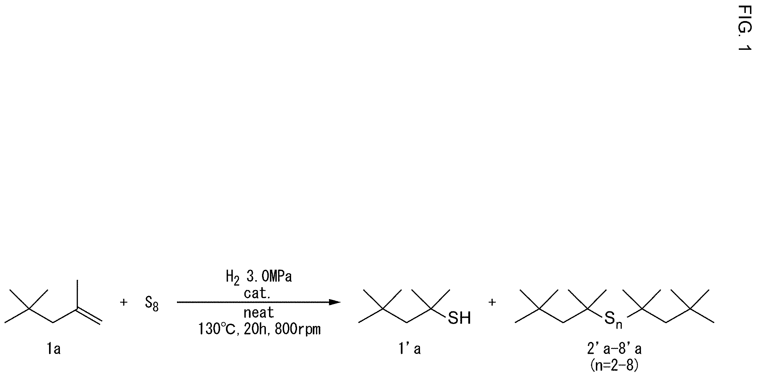

A stirrer, 20 mmol of diisobutylene (2,4,4-trimethyl-1-pentene), 0.1875 molar equivalent of powdered sulfur (S₈) (1.5 molar equivalents as S element), and 5 mol% of Co₃O₄ (manufactured by Sigma-Aldrich Co. LLC, nano powder: 50 nm or less (TEM)) were charged into an autoclave, and thereafter hydrogen was charged by pressurizing to 3.0 MPa. The autoclave was heated to 130°C while the resultant mixture was being stirred at 800 rpm using a magnetic stirrer, and a reaction was performed at the same temperature for 20 hours. Thereafter, the autoclave was cooled to room temperature and a pressure valve was opened. Thereafter, 200 mg of 1,3,5-tri-tert-butylbenzene as an internal standard (8.91 parts by mass relative to 100 parts by mass of diisobutylene) was added to the reaction solution. Further, air was blown into the autoclave to remove residual hydrogen sulfide. After removing unreacted sulfur and Co₃O₄ by a centrifugal separator, dialkyl polysulfide (A) was obtained. The reaction in the production method of the dialkyl polysulfide in Example 1 is illustrated in FIG. 1.

### (Example 2)

Dialkyl polysulfide (A) was obtained in the same manner as the manner in Example 1 except that the type of the metal catalyst was changed from Co₃O₄ to Co₃S₄. Co₃S₄ was obtained by adjusting according to a literature "Liu, Q.; Zhang, J. CrystEngComm, 2013, 15, 5087".

### (Example 3)

Dialkyl polysulfide (A) was obtained in the same manner as the manner in Example 1 except that the type of the metal catalyst was changed from Co₃O₄ to Ag₂O (manufactured by Wako Pure Chemical Corporation, Special Grade, Catalog Number 199-00882) .

### (Example 4)

Dialkyl polysulfide (A) was obtained in the same manner as the manner in Example 1 except that the type of the metal catalyst was changed from Co₃O₄ to MoO₃ (manufactured by Wako Pure Chemical Corporation, First Grade, Catalog Number 138-03352) .

### (Example 5)

Dialkyl polysulfide (A) was obtained in the same manner as the manner in Example 1 except that the type of the metal catalyst was changed from Co₃O₄ to CuO (manufactured by Kanto Chemical Co., Inc., Special Grade, Catalog Number 07503-30).

### (Example 6)

Dialkyl polysulfide (A) was obtained in the same manner as the manner in Example 1 except that the type of the metal catalyst was changed from Co₃O₄ to NiO (manufactured by Wako Pure Chemical Corporation, Catalog Number 140-01552, 99% or higher).

### (Example 7)

Dialkyl polysulfide (A) was obtained in the same manner as the manner in Example 1 except that the type of the metal catalyst was changed from Co₃O₄ to Fe₂O₃ (manufactured by Wako Pure Chemical Corporation, First Grade, Catalog Number 096-04825).

### (Example 8)

Dialkyl polysulfide (A) was obtained in the same manner as the manner in Example 1 except that the type of the metal catalyst was changed from Co₃O₄ to CuS (manufactured by FUJIFILM Wako Pure Chemical Corporation, Catalog Number 034-04462, 90% or more).

### (Example 9)

Dialkyl polysulfide (A) was obtained in the same manner as the manner in Example 1 except that the type of the metal catalyst was changed from Co₃O₄ to MoS₂ (manufactured by Kanto Chemical Co., Inc., Catalog No. 25368-32, 98% or more).

The dialkyl polysulfides (A) obtained in Examples 1 to 9 were measured and evaluated by the following methods.

### [Measurement of Yield and Total Yield of Dialkyl Polysulfides Having Different Number of Sulfur Atoms]

An ultra-high performance liquid chromatograph (manufactured by Waters Corporation, ACQUITY UPLC H-Class system) was used to obtain a chart of the dialkyl polysulfide (A) under the following measurement conditions. The yield (%) of each of the dialkyl polysulfides (n = 2 to 8) having different numbers of sulfur atoms was calculated by the ratio of the peak areas corresponding to the dialkyl polysulfides of each n value, using 1,3,5-tri-tert-butylbenzene as an internal standard. The total yield of the dialkyl polysulfide (A) was determined to be the total of the yield of each of the dialkyl polysulfides having different numbers of sulfur atoms. The conversion ratio was calculated from the yield of diisobutylene. The yields of thiol and diisobutylene were calculated from the results of ¹H NMR measurements of the sample of the recovered reaction solution diluted with CDCl₃ solvent using a FT-NMR apparatus (manufactured by JEOL Ltd., JNM-ECA600). In this measurement, 1,3,5-tri-tert-butylbenzene was also used as the internal standard.

### [UPLC Measurement Conditions]

Eluent: H₂O/MeCN/iPrOH = 25/25/50, Flow rate: 0.5 mL/min, Column: kinetex-C8, 2.6 um, 150 mm × 2.1 mm
Detector: PDA
Detection wavelength: 210 nm to 800 nm
[NMR Measurement Conditions]
¹H-NMR: 600 MHz

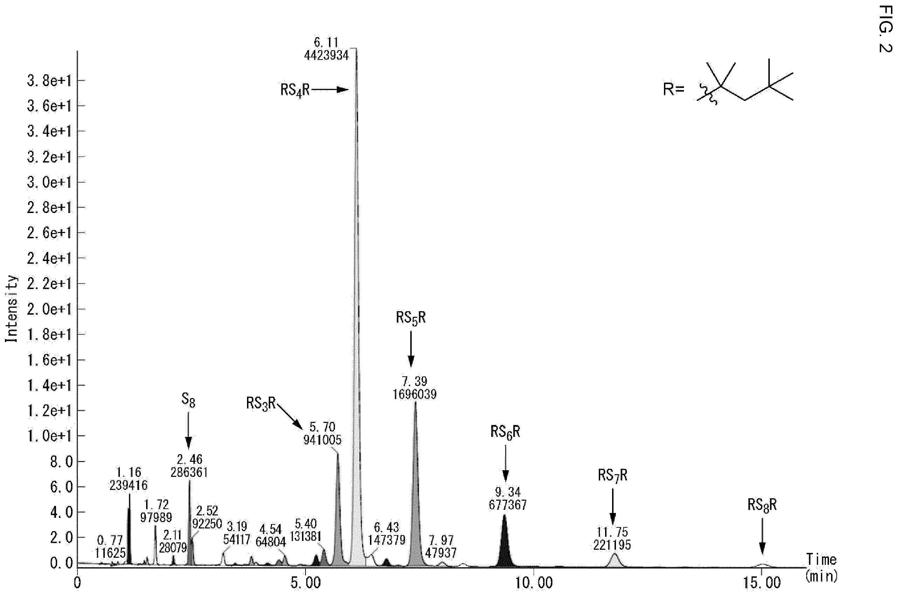

As an example, the chart of the dialkyl polysulfide (A) obtained in Example 1 and the peaks corresponding to the dialkyl polysulfides of each n value (n = 3 to 8) are illustrated in FIG. 2.

The measurement and evaluation results of Examples 1 to 9 are listed in Table 1. "N.D. (not determined)" in Table 1 indicates that the yield was not able to be calculated because the target peak was covered with other peaks.

**[Table 1]**

| Experimental number | Catalyst | Catalyst amount (mol%) | Na-X (mg) | Hydroge n pressur e (MPa) | S equ iva len t | Tern per atu re (°C) | Tim e (h) | Con ver sio n rat io | RSH yie ld | RS₂R yie ld | RS₃R yiel d | RS₄R yie ld | RS₅R yie ld | RSsR yie ld | RS₇R yie ld | RS₈R yie ld | RSₙR tot al yie ld |
|---|---|---|---|---|---|---|---|---|---|---|---|---|---|---|---|---|---|
| Example 3 | Ag₂O | 5 | - | 3.0 | 1.5 | 130 | 20 | 56 | 0 | 0 | N.D. | 3 | 3 | 1 | 0 | 0 | 7 |
| Example 4 | MoO₃ | 5 | - | 3.0 | 1.5 | 130 | 20 | 82 | 0 | 0 | N.D. | 2 | 5 | 1 | 0 | 0 | 8 |
| Example 5 | CuO | 5 | - | 3.0 | 1.5 | 130 | 20 | 72 | 0 | 0 | N.D. | 5 | 5 | 1 | 0 | 0 | 12 |
| Example 6 | NiO | 5 | - | 3.0 | 1.5 | 130 | 20 | 62 | 1 | 0 | N.D. | 5 | 10 | 3 | 1 | 0 | 19 |
| Example 7 | Fe₂O₃ | 5 | - | 3.0 | 1.5 | 130 | 20 | 60 | 0 | 0 | N.D. | 14 | 7 | 2 | 1 | 0 | 24 |
| Example 1 | Co₃O₉ | 5 | - | 3.0 | 1.5 | 130 | 20 | 83 | 2 | 0 | 11 | 29 | 9 | 3 | 1 | 0 | 53 |
| Example 2 | Co₃S₉ | 5 | - | 3.0 | 1.5 | 130 | 20 | 82 | 0 | 0 | 13 | 30 | 10 | 3 | 1 | 0 | 57 |
| Example 8 | CuS | 5 | - | 3.0 | 1.5 | 130 | 20 | 53 | 0 | 0 | 0 | 3 | 2 | 1 | 0 | 0 | 6 |
| Example 9 | MoS₂ | 5 | - | 3.0 | 1.5 | 130 | 20 | 50 | 0 | 0 | 0 | 3 | 2 | 1 | 0 | 0 | 6 |

From the results listed in Table 1, it was found that in any of Examples 1 to 9, the reaction of diisobutylene with powdered sulfur in the presence of hydrogen and any one of Co₃O₄, Co₃S₄, Ag₂O, MoO₃, CuO, NiO, Fe₂O₃, CuS, and MoS₂ gave the dialkyl polysulfides (A) having total yields of 6% to 57%. In particular, it was found that in Examples 1 and 2, the reaction of diisobutylene with sulfur in the presence of hydrogen and cobalt oxide or cobalt sulfide using Co as the metal element allowed the total yield of the dialkyl polysulfides (A) (n = 2 to 8) to be more than 50%.

### (Example 10)

A stirrer, 20 mmol of diisobutylene, 0.375 molar equivalent of powdered sulfur (S₈) (3.0 molar equivalents as S element), 2.5 mol% of CoS₂, and 100 mg of zeolite (manufactured by NACALAI TESQUE, INC., Na-X type, a fine product formed by crushing molecular sieve 13X and sieving to a particle diameter of 125 um or less) (4.45 parts by mass relative to 100 parts by mass of diisobutylene) were charged into an autoclave, and thereafter hydrogen was charged by pressurizing to 3.0 MPa. The autoclave was heated to 130°C while the resultant mixture was being stirred at 800 rpm using a magnetic stirrer, and a reaction was performed at the same temperature for 20 hours. Thereafter, the autoclave was cooled to room temperature and a pressure valve was opened. Thereafter, 200 mg of 1,3,5-tri-tert-butylbenzene as an internal standard (8.91 parts by mass relative to 100 parts by mass of diisobutylene) was added to the reaction solution. Further, air was blown into the autoclave to remove residual hydrogen sulfide. After removing unreacted sulfur, CoS₂, and zeolite by a centrifugal separator, dialkyl polysulfide (A) was obtained.

### (Example 11)

Dialkyl polysulfide (A) was obtained in the same manner as the manner in Example 10 except that the type of the metal catalyst was changed from CoS₂ to Co₃S₄. As Co₃S₄, the same Co₃S₄ used in Example 2 was used.

### (Example 12)

Dialkyl polysulfide (A) was obtained in the same manner as the manner in Example 10 except that the type of the metal catalyst was changed from CoS₂ to CoS (manufactured by Strem Chemicals, Inc.).

### (Example 13)

Dialkyl polysulfide (A) was obtained in the same manner as the manner in Example 10 except that the type of the metal catalyst was changed from CoS₂ to Co₃O₄ (manufactured by Sigma-Aldrich Co. LLC, nano powder: 50 nm or less (TEM)). The measurement and evaluation results of Examples 10 to 13 are listed in Table 2.

**[Table 2]**

| Experimental number | Catalyst | Catalyst amount (mol%) | Na-X (mg) | Hydroge n pressur e (MPa) | S equ iva len t | Tern per atu re (°C) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 12 | CoS | 2.5 | 100 | 3.0 | 3 | 130 | 20 | 51 | 0 | 0 | N.D. | 8 | 7 | 2 | 0 | 0 | 17 |
| Example 10 | CoS₂ | 2.5 | 100 | 3.0 | 3 | 130 | 20 | 97 | 1 | 0 | 12 | 41 | 19 | 7 | 2 | 1 | 82 |
| Example 11 | Co₃S₄ | 2.5 | 100 | 3.0 | 3 | 130 | 20 | 96 | 1 | 0 | 12 | 39 | 21 | 8 | 2 | 1 | 83 |
| Example 13 | Co₃O₄ | 2.5 | 100 | 3.0 | 3 | 130 | 20 | 90 | 1 | 1 | 8 | 22 | 24 | 8 | 2 | 1 | 64 |

From the results listed in Table 2, it was found that in any of Examples 10 to 13, the reaction of diisobutylene with powdered sulfur in the presence of hydrogen and any one of CoS, CoS₂, Co₃S₄, and Co₃O₄ gave the dialkyl polysulfides (A) in total yields of 17% to 83%. In particular, it was found that in Examples 10 and 11, the reaction of diisobutylene with sulfur in the presence of hydrogen, either CoS₂ (cobalt disulfide) or Co₃S₄ (tricobalt tetrasulfide), and the zeolite allowed the total yield of the dialkyl polysulfides (A) (n = 2 to 8) to be more than 80%. It was also found that in Example 11, the use of zeolite in addition to Co₃S₄ allowed the yield of the dialkyl polysulfide (A) to be improved as compared to the yield in Example 2.

### (Example 14)

Dialkyl polysulfide (A) was obtained in the same manner as the manner in Example 10 except that the charged amount of the zeolite (manufactured by NACALAI TESQUE, INC., Na-X type, a fine product formed by crushing molecular sieve 13X and sieving to a particle diameter of 125 um or less) was changed from 100 mg to 50 mg (2.23 parts by mass relative to 100 parts by mass of diisobutylene).

### (Example 15)

Dialkyl polysulfide (A) was obtained in the same manner as the manner in Example 10 except that the charged amount of the zeolite (manufactured by NACALAI TESQUE, INC., Na-X type, a fine product formed by crushing molecular sieve 13X and sieving to a particle diameter of 125 um or less) was changed from 100 mg to 200 mg (8.91 parts by mass relative to 100 parts by mass of diisobutylene). The measurement and evaluation results of Examples 14 and 15 and Example 10 as a reference are listed in Table 3.

**[Table 3]**

| Experimental number | Catalyst | Catalyst amount (mol%) | Na-X (mg) | Hydroge n pressur e (MPa) | S equ iva len t | Tern per atu re (°C) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 10 | CoS₂ | 2.5 | 100 | 3.0 | 3 | 130 | 20 | 97 | 1 | 0 | 12 | 41 | 19 | 7 | 2 | 1 | 82 |
| Example 14 | CoS₂ | 2.5 | 50 | 3.0 | 3 | 130 | 20 | 71 | 0 | 0 | 8 | 11 | 11 | 3 | 1 | 0 | 34 |
| Example 15 | CoS₂ | 2.5 | 200 | 3.0 | 3 | 130 | 20 | 96 | 0 | 0 | 9 | 34 | 18 | 7 | 2 | 1 | 70 |

From the results listed in Table 3, it was found that in both Example 14 and Example 15, the total yields of the dialkyl polysulfides (A) (n = 2 to 8) were more than 34%.

It was also found that when the charged amount of the zeolite was increased, the total yield of the dialkyl polysulfides (A) (n = 2 to 8) was increased and when the charged amount of the zeolite exceeded a certain amount, the total yield of the dialkyl polysulfides (A) (n = 2 to 8) tended to gradually decrease.

### (Example 16)

Dialkyl polysulfide (A) was obtained in the same manner as the manner in Example 10 except that the heating temperature of the autoclave, that is, a reaction temperature was changed from 130°C to 115°C.

### (Example 17)

Dialkyl polysulfide (A) was obtained in the same manner as the manner in Example 10 except that the heating temperature of the autoclave, that is, a reaction temperature was changed from 130°C to 115°C. The measurement and evaluation results of Examples 16 and 17 and Example 10 as a reference are listed in Table 4.

**[Table 4]**

| Experimental number | Catalyst | Catalyst amount (mol%) | Na-X (mg) | Hydroge n pressur e (MPa) | S equ iva len t | Tern per atu re (°C) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 10 | CoS₂ | 2.5 | 100 | 3.0 | 3 | 130 | 20 | 97 | 1 | 0 | 12 | 41 | 19 | 7 | 2 | 1 | 82 |
| Example 16 | CoS₂ | 2.5 | 100 | 3.0 | 3 | 115 | 20 | 31 | 0 | 0 | 2 | 4 | 3 | 1 | 0 | 0 | 10 |
| Example 17 | CoS₂ | 2.5 | 100 | 3.0 | 3 | 150 | 20 | 94 | 2 | 0 | 6 | 25 | 11 | 4 | 1 | 0 | 47 |

From the results listed in Table 4, it was found that in both Examples 16 and 17, the total yields of the dialkyl polysulfides (A) (n = 2 to 8) were more than 10%.

It was also found that in the range of the reaction temperature of 100°C to 160°C, when the reaction temperature became higher, the total yield of the dialkyl polysulfides (A) (n = 2 to 8) was increased and when the reaction temperature exceeded a certain value, the total yield of the dialkyl polysulfides (A) (n = 2 to 8) tended to gradually decrease.

### (Example 18)

A stirrer, 10 mmol of diisobutylene, 0.375 molar equivalent of powdered sulfur (S₈) (3.0 molar equivalents as S element), 2.5 mol% of CoS₂, and 50 mg of zeolite (manufactured by NACALAI TESQUE, INC., Na-X type, a fine product formed by crushing molecular sieve 13X and sieving to a particle diameter of 125 um or less) (4.45 parts by mass relative to 100 parts by mass of diisobutylene) were charged into an autoclave, and thereafter hydrogen was charged by pressurizing to 0.7 MPa. The autoclave was heated to 130°C while the resultant mixture was being stirred at 800 rpm using a magnetic stirrer, and a reaction was performed at the same temperature for 20 hours. Thereafter, the autoclave was cooled to room temperature and a pressure valve was opened. Thereafter, 100 mg of 1,3,5-tri-tert-butylbenzene as an internal standard (8.91 parts by mass relative to 100 parts by mass of diisobutylene) was added to the reaction solution. Further, air was blown into the autoclave to remove residual hydrogen sulfide. After removing unreacted sulfur, CoS₂, and zeolite by a centrifugal separator, dialkyl polysulfide (A) was obtained. Here, in this Example, due to the significant change in experimental conditions by a decrease in the pressure of hydrogen, the charged amount of the zeolite (Na-X) in this Example was set to 50 mg (1/2 scale) to perform the reaction.

### (Example 19)

Dialkyl polysulfide (A) was obtained in the same manner as the manner in Example 10 except that the hydrogen pressure during the charge was changed from 3.0 MPa to 5.0 MPa. The measurement and evaluation results of Examples 18 and 19 and Example 10 as a reference were listed in Table 5.

**[Table 5]**

| Experimental number | Catalyst | Catalyst amount (mol%) | Na-X (mg) | Hydroge n pressur e (MPa) | S equ iva len t | Tern per atu re (°C) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 10 | CoS₂ | 2.5 | 100 | 3.0 | 3 | 130 | 20 | 97 | 1 | 0 | 12 | 41 | 19 | 7 | 2 | 1 | 82 |
| Example 18 | CoS₂ | 2.5 | 50 | 0.7 | 3 | 130 | 20 | 69 | 0 | 0 | 6 | 9 | 7 | 2 | 1 | 0 | 25 |
| Example 19 | CoS₂ | 2.5 | 100 | 5.0 | 3 | 130 | 20 | 93 | 0 | 0 | 9 | 22 | 30 | 10 | 3 | 1 | 75 |

From the results listed in Table 5, it was found that in both Example 18 and Example 19, the total yields of the dialkyl polysulfides (A) (n = 2 to 8) were more than 25%.

It was also found that in the range of the hydrogen pressure of 1.0 MPa to 6.0 MPa, when the hydrogen pressure became higher, the total yield of the dialkyl polysulfides (A) (n = 2 to 8) was increased and when the hydrogen pressure exceeded a certain value, the total yield of the dialkyl polysulfides (A) (n = 2 to 8) tended to gradually decrease.

### (Example 20)

Dialkyl polysulfide (A) was obtained in the same manner as the manner in Example 10 except that the type of zeolite was changed to Na-X type (beads, Na-X type used in Example 10, an uncrushed product of molecular sieve 13X, grain diameter: about 2 mm).

### (Example 21)

Dialkyl polysulfide (A) was obtained in the same manner as the manner in Example 10 except that the type of zeolite was changed from Na-X type to Na-A type (manufactured by Wako Pure Chemical Corporation, catalog number 137-06085, molecular sieve 4A 1/16 was crushed and sieved to a particle diameter of 125 um or less).

### (Example 22)

Dialkyl polysulfide (A) was obtained in the same manner as the manner in Example 10 except that the type of zeolite was changed from Na-X type to K-A type (manufactured by Wako Pure Chemical Corporation, catalog number 134-06095, molecular sieve 3A 1/16 was crushed and sieved to a particle diameter of 125 um or less). The measurement and evaluation results of Examples 20 and 22 and Example 10 as a reference were listed in Table 6.

**[Table 6]**

| Experimental number | Catalyst | Catalys t amount (mol%) | Zeolite (100 mg) | Hydr ogen pres sure (MPa ) | S equ iva len t | Tern per atu re (°C ) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 10 | CoS₂ | 2.5 | Na-X | 3 | 3 | 130 | 20 | 97 | 1 | 0 | 12 | 41 | 19 | 7 | 2 | 1 | 82 |
| Example 20 | CoS₂ | 2.5 | Na-X (Beads) | 3 | 3 | 130 | 20 | 72 | 0 | 0 | 9 | 6 | 9 | 3 | 1 | 0 | 28 |
| Example 21 | CoS₂ | 2.5 | Na-A | 3 | 3 | 130 | 20 | 76 | 0 | 0 | 8 | 9 | 9 | 3 | 1 | 0 | 30 |
| Example 22 | CoS₂ | 2.5 | K-A | 3 | 3 | 130 | 20 | 83 | 0 | 0 | 8 | 20 | 13 | 4 | 1 | 0 | 46 |

From the results listed in Table 6, it was found that in any of Examples 20 to 22, the total yields of the dialkyl polysulfides (A) (n = 2 to 8) were more than 28%.

It was found that when the Na-X type and the Na-A type having the same cation and different pore structures were compared, the total yield of the dialkyl polysulfides (A) (n = 2 to 8) when the zeolite having a pore structure of the X type was used was higher than the total yield when the zeolite having a pore structure of the A type. It was also found that among the Na-X types, the total yield of the dialkyl polysulfides (A) (n = 2 to 8) was higher when the Na-X type having a smaller particle diameter by crashing was used. Furthermore, when the Na-A type was compared to the K-A type having the same pore structure and different cations, it was found that the total yield of the dialkyl polysulfides (A) (n = 2 to 8) was higher when the cation was K ion than the total yield when the cation was Na ion.

### (Example 23)

A stirrer, 20 mmol of diisobutylene, and 0.1875 molar equivalent of powdered sulfur (S₈) (1.5 molar equivalents as S element) were charged into an autoclave, and thereafter hydrogen was charged by pressurizing to 3.0 MPa. The autoclave was heated to 130°C while the resultant mixture was being stirred at 800 rpm using a magnetic stirrer, and a reaction was performed at the same temperature for 20 hours. Thereafter, the autoclave was cooled to room temperature and a pressure valve was opened. Thereafter, 200 mg of 1,3,5-tri-tert-butylbenzene as an internal standard (8.91 parts by mass relative to 100 parts by mass of diisobutylene) was added to the reaction solution. Further, air was blown into the autoclave to remove residual hydrogen sulfide. After removing unreacted sulfur by a centrifugal separator, dialkyl polysulfide (A) was obtained.

### (Example 24)

A stirrer, 20 mmol of diisobutylene, 0.1875 molar equivalent of powdered sulfur (S₈) (1.5 molar equivalents as S element), and 100 mg of zeolite (manufactured by NACALAI TESQUE, INC., Na-X type, a fine product formed by crushing molecular sieve 13X and sieving to a particle diameter of 125 um or less) were charged into an autoclave, and thereafter hydrogen was charged by pressurizing to 3.0 MPa. The autoclave was heated to 130°C while the resultant mixture was being stirred at 800 rpm using a magnetic stirrer, and a reaction was performed at the same temperature for 20 hours. Thereafter, the autoclave was cooled to room temperature and a pressure valve was opened. Thereafter, 200 mg of 1,3,5-tri-tert-butylbenzene as an internal standard (8.91 parts by mass relative to 100 parts by mass of diisobutylene) was added to the reaction solution. Further, air was blown into the autoclave to remove residual hydrogen sulfide. After removing unreacted sulfur and the zeolite by a centrifugal separator, dialkyl polysulfide (A) was obtained. The measurement and evaluation results of Examples 23 and 24 and Example 1 as a reference were listed in Table 7.

**[Table 7]**

| Experimental number | Catalyst | Catalyst amount (mol%) | Na-X (mg) | Hydroge n pressur e (MPa) | S equ iva len t | Tern per atu re (°C ) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 1 | Co₃O₄ | 5 | - | 3.0 | 1.5 | 130 | 20 | 83 | 2 | 0 | 11 | 29 | 9 | 3 | 1 | 0 | 53 |
| Example 23 | - | - | - | 3.0 | 1.5 | 130 | 20 | 57 | 0 | 0 | 3 | 8 | 2 | 1 | 0 | 0 | 14 |
| Example 24 | - | - | 100 | 3.0 | 1.5 | 130 | 20 | 35 | 0 | 0 | 4 | 2 | 4 | 1 | 0 | 0 | 11 |

From the results in Table 7, it was found that in Example 23, the reaction of diisobutylene with powdered sulfur in the presence of hydrogen gave dialkyl polysulfide (A) in a total yield of 14%. In addition, it was found that, in Example 24, the reaction of diisobutylene with powdered sulfur in the presence of hydrogen and zeolite gave dialkyl polysulfide (A) in a total yield of 11%.

### (Comparative Example 1)

Into a 1-liter autoclave equipped with a heating device, hydrogen sulfide blowpipe, and hydrogen sulfide absorption equipment, 365 g (3.26 mol) of diisobutylene, 104 g (3.26 mol) of powdered sulfur, 0.1 g of potassium hydroxide, and 4 g of butylcarbitol were charged. The autoclave was tightly closed, and thereafter a pressure in the reaction container was reduced to -0.1 MPa or less using a vacuum pump to perform vacuum degassing. Thereafter, the autoclave was tightly closed and thereafter heated until the internal temperature reached 120°C. Into the autoclave, 65 g (1.9 mol) of hydrogen sulfide gas (purity 99.9 mol%) was blown at a pressure of 6 kg/cm² over a period of 20 hours. Furthermore, the autoclave was maintained at the same temperature for 10 hours. Thereafter, the autoclave was cooled to 40°C, the valve connected to the hydrogen sulfide absorption equipment was opened to return the pressure to normal pressure, and air was blown through the blowpipe to remove residual hydrogen sulfide and unreacted diisobutylene to give dialkyl polysulfide (A) in a total yield of 87%.

Although the dialkyl polysulfide (A) was obtained in high yield in Comparative Example 1, hydrogen sulfide gas is an essential material, which is significantly different from the production methods in Examples 1 to 24 using hydrogen an essential material. In addition, the basic compound is used as a catalyst in Comparative Example 1. It is found that the catalyst used in Comparative Example 1 is different from the metal elements and zeolites used as catalysts in Examples 1 to 22.

### (Comparative Example 2)

In a 1-liter autoclave equipped with a heating device, hydrogen sulfide blowpipe, and hydrogen sulfide absorption equipment, 786 g (7.0 mol) of diisobutylene, 379 g (11.8 mol) of powdered sulfur, and 2.3 g of dicyclohexylamine as a catalyst were charged. Thereafter, the autoclave was tightly closed and heated until the internal temperature reached 120°C. Into the autoclave, 129 g (3.8 mol) of hydrogen sulfide gas (purity 99.9 mol%) was blown at a pressure of 6 kg/cm² over a period of time of 20 hours. Furthermore, the autoclave was maintained at the same temperature for 1 hours. Thereafter, the autoclave was cooled to 70°C, the valve connected to the hydrogen sulfide absorption equipment was opened to return the pressure to normal pressure, and air was blown through the blowpipe to remove residual hydrogen sulfide and unreacted diisobutylene to give dialkyl polysulfide (A) in a total yield of 90%.

Although the dialkyl polysulfide (A) was obtained in high yield in Comparative Example 2, hydrogen sulfide gas is the essential material similar to Comparative Example 1, and thus this production method is significantly different from the production methods in Examples 1 to 24 using hydrogen as the essential material. In addition, the amine-based catalyst is used in Comparative Example 2 and thus it is found that the catalyst used in Comparative Example 2 is different from the metal elements and zeolites used as the catalysts in Examples 1 to 22.

### (Example 25)

Dialkyl polysulfide (A) was obtained in the same manner as the manner in Example 1 except that the amount of powdered sulfur (S₈) was changed from 1.5 molar equivalents to 3.0 molar equivalents as the S element.

### (Example 26)

Dialkyl polysulfide (A) was obtained in the same manner as the manner in Example 25 except that the type of the metal catalyst was changed from Co₃O₄ to CoₓS_{y} (including Co₃O₄ and having a Co content of 50% by mass) and another zeolite (manufactured by Sigma-Aldrich Co. LLC, Na-X type, particle diameter: less than 2 um, a commercial product was used as it was without performing hydration treatment) was used. CoₓS_{y} was obtained by adjusting according to a literature "Qiao Liu, Junyan Zhang, CrystEngComm, 2013, 15, 5087".

### (Example 27)

Dialkyl polysulfide (A) was obtained in the same manner as the manner in Example 25 except that the type of the metal catalyst was changed from Co₃O₄ to CoₓS_{y} (including Co₃O₄ and having a Co content of 45% by mass) and another zeolite (manufactured by Sigma-Aldrich Co. LLC, Na-X type, particle diameter: less than 2 um, a commercial product was used as it was without performing hydration treatment) was used.

### (Example 28)

Dialkyl polysulfide (A) was obtained in the same manner as the manner in Example 25 except that another zeolite (manufactured by Sigma-Aldrich Co. LLC, Na-X type, particle diameter: less than 2 um, commercial product was used as it was without performing hydration treatment) was used. The results are listed in Table 8.

**[Table 8]**

| Experimental number | Catalyst | Catalys t amount (mol%) | Na-X (mg) | Hydroge n pressur e (MPa) | S equ iva len t | Tern per atu re (°C ) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 25 | Co₃O₄ | 5.0 | - | 3.0 | 3.0 | 130 | 20 | 95 | 0 | 0 | 9 | 32 | 16 | 6 | 2 | 1 | 65 |
| Example 26 | COₓS_{y} [TKK119] | 5.0 | 100 | 5.0 | 3.0 | 130 | 20 | 97 | 3 | 0 | 11 | 35 | 24 | 8 | 2 | 1 | 81 |
| Example 27 | COₓS_{y} [TKK143] | 5.0 | 100 | 5.0 | 3.0 | 130 | 20 | 97 | 7 | 0 | 14 | 35 | 16 | 5 | 1 | 0 | 72 |
| Example 28 | Co₃O₄ | 5.0 | 100 | 5.0 | 3.0 | 130 | 20 | 98 | 5 | 0 | 12 | 40 | 20 | 7 | 2 | 1 | 83 |

From the results listed in Table 8, it was found that in Example 28, in the case where the type of metal catalyst was Co₃O₄, the total yield of dialkyl polysulfides (A) (n = 2 to 8) was 83%, which was a similar total yield to the total yield in the case where the type of metal catalyst was CoₓO_{y} (Examples 26 and 27). It was also found that the use of Na-X type zeolite in the case where the metal catalyst type was Co₃O₄ allowed a higher total yield to be obtained as compared to the total yield in the case where the Na-X type zeolite was not used (Example 25).

### (Example 29)

Dialkyl polysulfide (A) was obtained in the same manner as the manner in Example 13 except that the amount of catalyst was changed from 2.5 mol% to 5.0 mol%, the hydrogen pressure was changed from 3.0 MPa to 0.7 MPa, and another zeolite (manufactured by Sigma-Aldrich, Na-X type, particle diameter: less than 2 um, commercial product with hydration treatment (water content: 24% by mass)) was used.

### (Example 30)

Dialkyl polysulfide (A) was obtained in the same manner as the manner in Example 29 except that the hydrogen pressure was changed from 0.7 MPa to 3.0 MPa.

### (Example 31)

Dialkyl polysulfide (A) was obtained in the same manner as the manner in Example 29 except that the hydrogen pressure was changed from 0.7 MPa to 5.0 MPa and the amount of powdered sulfur (S₈) was changed from 3.0 molar equivalents to 1.5 molar equivalents as the S element.

### (Example 32)

Dialkyl polysulfide (A) was obtained in the same manner as the manner in Example 29 except that the hydrogen pressure was changed from 0.7 MPa to 5.0 MPa and the amount of powdered sulfur (S₈) was changed from 3.0 molar equivalents to 5.0 molar equivalents as the S element.

### (Example 33)

Dialkyl polysulfide (A) was obtained in the same manner as the manner in Example 29 except that the hydrogen pressure was changed from 0.7 MPa to 5.0 MPa and the reaction time was changed from 20 hours to 2 hours.

### (Example 34)

Dialkyl polysulfide (A) was obtained in the same manner as the manner in Example 33 except that the Na-X type zeolite was not used.

### (Example 35)

Dialkyl polysulfide (A) was obtained in the same manner as the manner in Example 29 except that the hydrogen pressure was changed from 0.7 MPa to 5.0 MPa and the heating temperature (reaction temperature) was changed from 130°C to 115°C.

### (Example 36)

Dialkyl polysulfide (A) was obtained in the same manner as the manner in Example 35 except that the heating temperature was changed from 130°C to 150°C.

### (Example 37)

Dialkyl polysulfide (A) was obtained in the same manner as the manner in Example 29 except that the hydrogen pressure was changed from 0.7 MPa to 5.0 MPa. The results are listed in Table 9.

**[Table 9]**

| Experimental number | Catalyst | Catalyst amount (mol%) | Na-X (mg) | Hydroge n pressur e (MPa) | S equ iva len t | Tern per atu re (°C ) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 29 | Co₃O₄ | 5.0 | 100 | 0.7 | 3.0 | 130 | 20 | 89 | 0 | 0 | 6 | 10 | 16 | 6 | 2 | 0 | 41 |
| Example 30 | Co₃O₄ | 5.0 | 100 | 3.0 | 3.0 | 130 | 20 | 97 | 3 | 0 | 11 | 40 | 19 | 7 | 2 | 1 | 80 |
| Example 31 | Co₃O₄ | 5.0 | 100 | 5.0 | 1.5 | 130 | 20 | 83 | 47 | 3 | 12 | 0 | 0 | 0 | 0 | 0 | 15 |
| Example 32 | Co₃O₄ | 5.0 | 100 | 5.0 | 5.0 | 130 | 20 | 99 | 3 | 0 | 6 | 36 | 22 | 8 | 3 | 1 | 76 |
| Example 33 | Co₃O₄ | 5.0 | 100 | 5.0 | 3.0 | 130 | 2 | 27 | 0 | 0 | 1 | 3 | 5 | 2 | 0 | 0 | 12 |
| Example 34 | Co₃O₄ | 5.0 | - | 5.0 | 3.0 | 130 | 2 | 15 | 0 | 0 | 1 | 2 | 2 | 1 | 0 | 0 | 6 |
| Example 35 | Co₃O₄ | 5.0 | 100 | 5.0 | 3.0 | 115 | 20 | 82 | 1 | 0 | 0 | 33 | 19 | 7 | 2 | 1 | 62 |
| Example 36 | Co₃O₄ | 5.0 | 100 | 5.0 | 3.0 | 150 | 20 | 87 | 48 | 4 | 10 | 4 | 0 | 0 | 0 | 0 | 17 |
| Example 37 | Co₃O₄ | 5.0 | 100 | 5.0 | 3.0 | 130 | 20 | 97 | 4 | 0 | 13 | 34 | 24 | 11 | 4 | 2 | 88 |

From the results listed in Table 9, it was found that in Example 29, the total yield of the dialkyl polysulfides (A) (n = 2 to 8) was 41% when the hydrogen pressure was 0.7 MPa and thus an excellent total yield was obtained. It was also found that in Examples 30 and 37, the total yields of dialkyl polysulfides (A) (n = 2 to 8) were 80% and 88%, respectively, and thus even better total yields were obtained when the hydrogen pressure was 3.0 MPa and 5.0 MPa.

In Example 31, when the S equivalent was 1.5 molar equivalents, thiol was a main product and the total yield of the dialkyl polysulfides (A) (n = 2 to 8) was 15%. It was found that in Example 37, the total yield of the dialkyl polysulfides (A) (n = 2 to 8) was 88% when the S equivalent was 3.0 molar equivalents, and the total yield was extremely better than the total yield when the S equivalent was 1.5 molar equivalents (Example 31). It was found that in Example 32, the total yield of the dialkyl polysulfides (A) (n = 2 to 8) was 76% when the S equivalent was 5.0 molar equivalents. Although the total yield decreased as compared to the total yield when the S equivalent was 3.0 molar equivalents (Example 37), the total yield was increased as compared to the total yield when the S equivalent was 1.5 molar equivalents (Example 31).

It was found that in Example 33, the total yield of the dialkyl polysulfides (A) (n = 2 to 8) was 12% when the reaction time was 2 hours. The total yield was lowered than the total yield when the reaction time was 20 hours (Example 29). A similar tendency was observed in Example 34, in which no zeolite was used.

In addition, it was found that in Example 35, the total yield of the dialkyl polysulfides (A) (n = 2 to 8) was 62% when the heating temperature (reaction temperature) was 115°C and thus the total yield was lowered as compared to the total yield when the heating temperature (reaction temperature) was 130°C (Example 37). It was found that in Example 36, the total yield of the dialkyl polysulfides (A) (n = 2 to 8) was 17% when the heating temperature (reaction temperature) was 150°C because thiol was the main product and thus the yield was lowered as compared to the total yields when the heating temperatures (reaction temperature) were 130°C (Example 37) and 150°C (Example 35).

### (Example 38)

Dialkyl polysulfide (A) was obtained in the same manner as the manner in Example 37 except that the charged amount of the zeolite (manufactured by Sigma-Aldrich, Na-X type, particle diameter: less than 2 um, commercial product with hydration treatment (water content: 24% by mass)) was changed from 100 mg to 50 mg.

### (Example 39)

Dialkyl polysulfide (A) was obtained in the same manner as the manner in Example 37 except that the charged amount of the zeolite (manufactured by Sigma-Aldrich, Na-X type, particle diameter: less than 2 um, commercial product with hydration treatment (water content: 24% by mass)) was changed from 100 mg to 200 mg.

### (Example 40)

Dialkyl polysulfide (A) was obtained in the same manner as the manner in Example 37 except that the catalyst used in Example 37 was reused (reused once) together with the zeolite. The results are listed in Table 10.

**[Table 10]**

| Experimental number | Catalyst | Catalyst amount (mol%) | Na-X (mg) | Hydroge n pressur e (MPa) | S equ iva len t | Tern per atu re (°C ) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 38 | Co₃O₄ | 5.0 | 50 | 5.0 | 3.0 | 130 | 20 | 97 | 5 | 0 | 13 | 30 | 26 | 11 | 4 | 1 | 85 |
| Example 39 | Co₃O₄ | 5.0 | 200 | 5.0 | 3.0 | 130 | 20 | 97 | 7 | 0 | 16 | 39 | 20 | 8 | 3 | 1 | 87 |
| Example 40 | Co₃O₄ | 5.0 | 100 | 5.0 | 3.0 | 130 | 20 | 97 | 5 | 0 | 14 | 39 | 23 | 9 | 3 | 1 | 88 |

From the results listed in Table 10, it was found that in Examples 38 and 39, the total yields of the dialkyl polysulfides (A) (n = 2 to 8) were 85% and 87%, respectively when the charged amounts of the zeolite were 50 mg and 200 mg and thus almost the same total yield as the total yield when the charged amount of the zeolite was 100 mg (Example 37) was obtained.

In addition, it was found that in Example 40, reuse of the catalyst together with the zeolite allowed almost the same total yield as the total yield when the catalyst was firstly used (Example 37) to be obtained.

### (Example 41)

Dialkyl polysulfide (A) was obtained in the same manner as the manner in Example 37 except that the raw material substrate was changed from 2,4,4-trimethyl-1-pentene to 2,4,4-trimethyl-2-pentene.

### (Example 42)

Dialkyl polysulfide (A) was obtained in the same manner as the manner in Example 37 except that the reaction time was changed from 20 hours to 12 hours.

### (Example 43)

Dialkyl polysulfide (A) was obtained in the same manner as the manner in Example 34 except that the reaction time was changed from 2 hours to 12 hours.

### (Example 44)

Dialkyl polysulfide (A) was obtained in the same manner as the manner in Example 37 except that the catalyst used in Example 37 was reused (reused two times) together with the zeolite.

### (Example 45)

Dialkyl polysulfide (A) was obtained in the same manner as the manner in Example 37 except that the reaction time was changed from 20 hours to 4 hours.

### (Example 46)

Dialkyl polysulfide (A) was obtained in the same manner as the manner in Example 34 except that the reaction time was changed from 2 hours to 4 hours.

### (Example 47)

Dialkyl polysulfide (A) was obtained in the same manner as the manner in Example 34 except that the reaction time was changed from 2 hours to 20 hours.

### (Example 48)

Dialkyl polysulfide (A) was obtained in the same manner as the manner in Example 37 except that the catalyst used in Example 37 was reused (reused three times) together with the zeolite.

### (Example 49)

Dialkyl polysulfide (A) was obtained in the same manner as the manner in Example 37 except that the hydrogen pressure was changed from 5.0 MPa to 3.0 MPa and the reaction time was changed from 20 hours to 2 hours.

### (Example 50)

Dialkyl polysulfide (A) was obtained in the same manner as the manner in Example 34 except that the hydrogen pressure was changed from 5.0 MPa to 3.0 MPa.

### (Example 51)

Dialkyl polysulfide (A) was obtained in the same manner as the manner in Example 37 except that the catalyst used in Example 37 was reused (reused four times) together with the zeolite. The results are listed in Table 11.

### (Example 52)

Dialkyl polysulfide (A) was obtained in the same manner as the manner in Example 37 except that the hydrogen pressure was changed from 5.0 MPa to 3.0 MPa and the reaction time was changed from 20 hours to 12 hours.

### (Example 53)

Dialkyl polysulfide (A) was obtained in the same manner as the manner in Example 34 except that the hydrogen pressure was changed from 5.0 MPa to 3.0 MPa and the reaction time was changed from 2 hours to 12 hours.

### (Example 54)

Dialkyl polysulfide (A) was obtained in the same manner as the manner in Example 52 except that the reaction time was changed from 12 hours to 4 hours.

### (Example 55)

Dialkyl polysulfide (A) was obtained in the same manner as the manner in Example 53 except that the reaction time was changed from 12 hours to 4 hours.

### (Comparative Example 3)

Dialkyl polysulfide (A) was obtained in the same manner as the manner in Example 37 except that the hydrogen pressure was changed from 5.0 MPa to 3.0 MPa and the S equivalent was changed from 3.0 molar equivalents to 0.

### (Comparative Example 4)

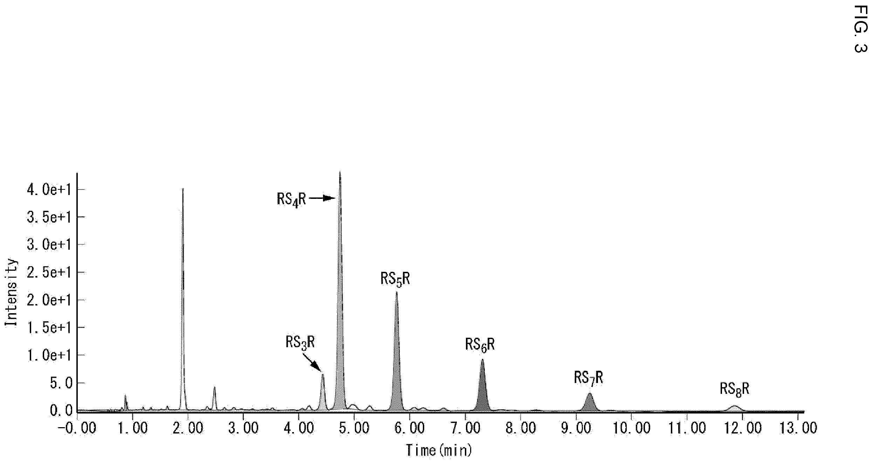

Dialkyl polysulfide (A) was obtained in the same manner as the manner in Example 34 except that the hydrogen pressure was changed from 5.0 MPa to 3.0 MPa, the S equivalent was changed from 3.0 molar equivalents to 0, and the reaction time was changed from 2 hours to 20 hours. The results are listed in Table 11 and illustrated in FIG. 3 to FIG. 8.

**[Table 11]**

| Experimental number | Catalyst | Catalyst amount (mol%) | Na-X (mg) | Hydroge n pressur e (MPa) | S equ iva len t | Tern per atu re (°C ) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 41 | Co₃O₄ | 5.0 | 100 | 5.0 | 3.0 | 130 | 20 | 99 | 6 | 0 | 13 | 37 | 18 | 8 | 3 | 1 | 80 |
| Example 42 | Co₃O₄ | 5.0 | 100 | 5.0 | 3.0 | 130 | 12 | 96 | 4 | 0 | 11 | 40 | 20 | 8 | 3 | 1 | 83 |
| Example 43 | Co₃O₄ | 5.0 | - | 5.0 | 3.0 | 130 | 12 | 94 | 3 | 0 | 8 | 36 | 22 | 9 | 3 | 1 | 79 |
| Example 44 | Co₃O₄ | 5.0 | 100 | 5.0 | 3.0 | 130 | 20 | 97 | 6 | 0 | 14 | 39 | 20 | 9 | 3 | 1 | 86 |
| Example 45 | Co₃O₄ | 5.0 | 100 | 5.0 | 3.0 | 130 | 4 | 46 | 0 | 0 | 3 | 12 | 9 | 3 | 1 | 0 | 29 |
| Example 46 | Co₃O₄ | 5.0 | - | 5.0 | 3.0 | 130 | 4 | 45 | 0 | 0 | 3 | 11 | 7 | 3 | 1 | 0 | 26 |
| Example 47 | Co₃O₄ | 5.0 | - | 5.0 | 3.0 | 130 | 20 | 97 | 6 | 0 | 14 | 41 | 18 | 7 | 2 | 1 | 83 |
| Example 48 | Co₃O₄ | 5.0 | 100 | 5.0 | 3.0 | 130 | 20 | 97 | 4 | 0 | 13 | 40 | 22 | 10 | 4 | 1 | 89 |
| Example 49 | Co₃O₄ | 5.0 | 100 | 3.0 | 3.0 | 130 | 2 | 21 | 0 | 0 | 1 | 2 | 3 | 2 | 1 | 0 | 9 |
| Example 50 | Co₃O₄ | 5.0 | - | 3.0 | 3.0 | 130 | 2 | 11 | 0 | 0 | 1 | 1 | 2 | 1 | 0 | 0 | 5 |
| Example 51 | Co₃O₄ | 5.0 | 100 | 5.0 | 3.0 | 130 | 20 | 98 | 5 | 0 | 14 | 42 | 20 | 9 | 3 | 1 | 89 |
| Example 52 | Co₃O₄ | 5.0 | 100 | 3.0 | 3.0 | 130 | 12 | 86 | 0 | 0 | 7 | 16 | 27 | 11 | 4 | 1 | 66 |
| Example 53 | Co₃O₄ | 5.0 | - | 3.0 | 3.0 | 130 | 12 | 82 | 0 | 0 | 7 | 17 | 24 | 10 | 3 | 1 | 63 |
| Example 54 | Co₃O₄ | 5.0 | 100 | 3.0 | 3.0 | 130 | 4 | 32 | 0 | 1 | 2 | 3 | 7 | 4 | 1 | 0 | 18 |
| Example 55 | Co₃O₄ | 5.0 | - | 3.0 | 3.0 | 130 | 4 | 25 | 0 | 1 | 2 | 3 | 5 | 3 | 1 | 0 | 14 |
| Comparative Example 3 | Co₃O₄ | 5.0 | 100 | 3.0 | 0.0 | 130 | 20 | 99> | 0 | 0 | 0 | 0 | 0 | 0 | 0 | 0 | 0 |
| Comparative Example 4 | Co₃O₄ | 5.0 | - | 3.0 | 0.0 | 130 | 20 | 99> | 0 | 0 | 0 | 0 | 0 | 0 | 0 | 0 | 0 |

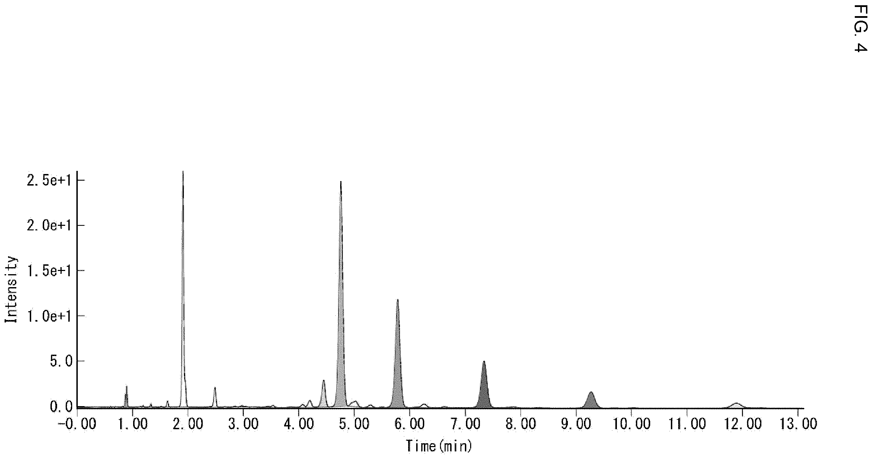

From the results listed in Table 11, it was found that in Examples 41, the total yield was 80% in the case where the substrate was 2,4,4-trimethyl-2-pentene and thus an excellent total yield was obtained even when the different raw material substrate was used. The chart of dialkyl polysulfide (A) obtained in Example 41 and the peaks corresponding to the dialkyl polysulfides of each n value (n = 3 to 8) are illustrated in FIG. 3 and the chart of the dialkyl polysulfide (A) obtained in Example 42 and the peaks corresponding to dialkyl polysulfides of each n value (n = 3 to 8) are illustrated in FIG. 4 as a reference.

It was found that in Examples 42 and 43, when the reaction time was 12 hours, the difference in conversion ratios caused by the presence or absence of the zeolite was small as compared to the case where the reaction time was 2 hours (Examples 33 and 34). It was confirmed that there was a similar tendency in the case where the reaction time was 20 hours.

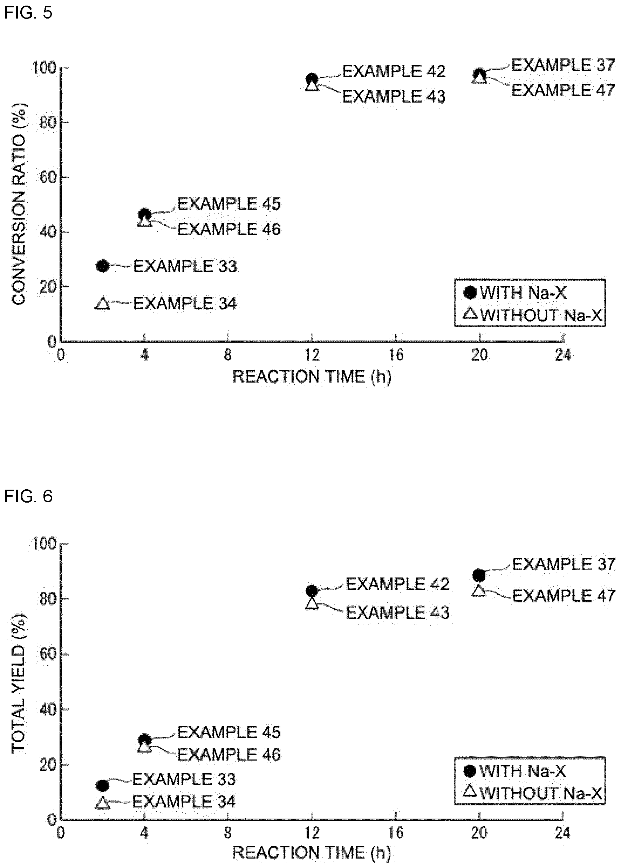

The relation between the reaction time and the conversion ratio in the case where the hydrogen pressure was set to 5.0 MPa is illustrated in FIG. 5. As illustrated in FIG. 5, it was confirmed that when the reaction time is 4 hours or more, the difference in the conversion ratios caused by the presence or absence of the zeolite is small, whereas when the reaction time is less than 4 hours, the difference in the conversion ratios caused by the presence or absence of the zeolite is large.

In addition, the relationship between the reaction time and the total yield in the case where the hydrogen pressure was set to 5.0 MPa is illustrated in FIG. 6. As illustrated in FIG. 6, it was confirmed that in contrast to the conversion ratio, the difference in the total yields caused by the presence or absence of the zeolite was small regardless of the reaction time.

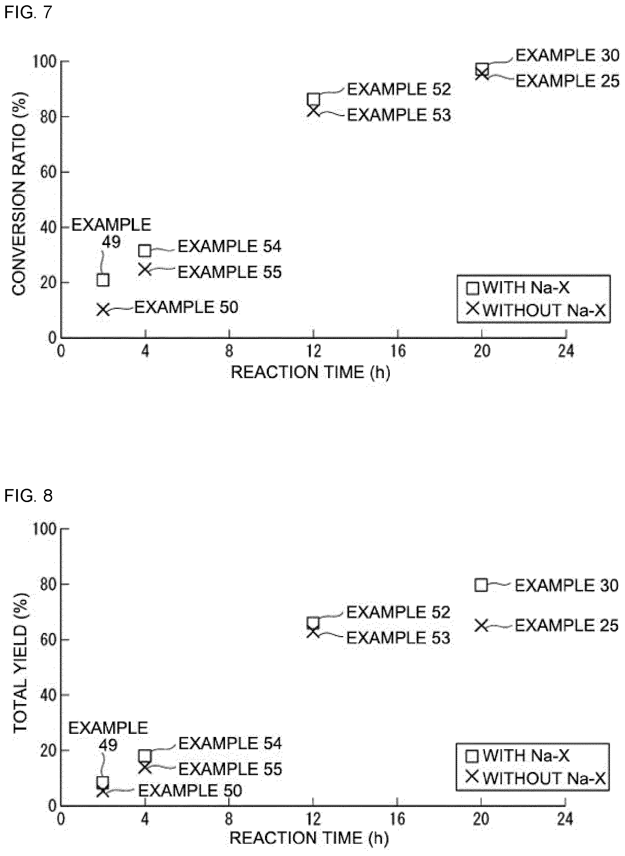

The relation between the reaction time and the conversion ratio in the case where the hydrogen pressure was set to 3.0 MPa is illustrated in FIG. 7. As illustrated in FIG. 7, it was confirmed that when the reaction time is 4 hours or more, the difference in the conversion ratios caused by the presence or absence of the zeolite is small, whereas when the reaction time is less than 4 hours, the difference in the conversion ratios caused by the presence or absence of the zeolite is large.

The relation between the reaction time and the total yield in the case where the hydrogen pressure was set to 3.0 MPa is illustrated in FIG. 8. As illustrated in FIG. 8, it was confirmed that the difference in the conversion ratios caused by the presence or absence of the zeolite was small when the reaction time was less than 20 hours, whereas the difference in the conversion ratios caused by the presence or absence of the zeolite was large when the reaction time was 20 hours or more.

It was found that in Examples 44, 48, and 51, when the catalyst was reused two times to four times together with the zeolite, almost the same total yields were obtained as the total yield when the catalyst was firstly used (Example 37) .

It was found that in Comparative Examples 3 and 4, when the S equivalent was set to 0, most of the substrate was used in the reaction regardless of the presence or absence of the Na-X type zeolite, and a reduced alkane (2,4,4-trimethylpentane) was produced.

### (Example 56)

Dialkyl polysulfide (A) was obtained in the same manner as the manner in Example 37 except that the catalyst used in Example 37 was reused (reused five times) together with the zeolite.

### (Example 57)

Dialkyl polysulfide (A) was obtained in the same manner as the manner in Example 37 except that the catalyst used in Example 37 was reused (reused six times) together with the zeolite.

### (Example 58)

Dialkyl polysulfide (A) was obtained in the same manner as the manner in Example 37 except that the catalyst used in Example 37 was reused (reused seven times) together with the zeolite.

### (Example 59)

Dialkyl polysulfide (A) was obtained in the same manner as the manner in Example 37 except that the hydrogen pressure was changed from 5.0 MPa to 7.0 MPa.

### (Example 60)

Dialkyl polysulfide (A) was obtained in the same manner as the manner in Example 37 except that the catalyst used in Example 37 was reused (reused eight times) together with the zeolite.

### (Example 61)

Dialkyl polysulfide (A) was obtained in the same manner as the manner in Example 37 except that the Na-X type zeolite was replaced with Na₂CO₃ (manufactured by Wako Pure Chemical Corporation, 5.0 mol% (1 mmol relative to 20 mmol of the substrate)) as an example of a basic compound and the hydrogen pressure was changed from 5.0 MPa to 3.0 MPa.

### (Example 62)

Dialkyl polysulfide (A) was obtained in the same manner as the manner in Example 37 except that the catalyst used in Example 37 was reused (reused nine times) together with the zeolite.

### (Example 63)

Dialkyl polysulfide (A) was obtained in the same manner as the manner in Example 37 except the charged amount of the zeolite (manufactured by Sigma-Aldrich Co. LLC, Na-X type, particle diameter: less than 2 um, commercial product with hydration treatment (water content: 24% by mass)) was changed from 100 mg to 500 mg and the hydrogen pressure was changed from 5.0 MPa to 3.0 MPa.

### (Example 64)

Dialkyl polysulfide (A) was obtained in the same manner as the manner in Example 37 except that the catalyst used in Example 37 was reused (reused ten times) together with the zeolite.

### (Example 65)

Dialkyl polysulfide (A) was obtained in the same manner as the manner in Example 37 except that the Na-X type zeolite was replaced with dicyclohexylamine (manufactured by Wako Pure Chemical Corporation, 5.0 mol% (1 mmol relative to 20 mmol of the substrate)) as an example of a basic compound and the hydrogen pressure was changed from 5.0 MPa to 3.0

MPa.

### (Example 66)

Dialkyl polysulfide (A) was obtained in the same manner as the manner in Example 37 except that the Na-X type zeolite was replaced with NaOH (manufactured by Wako Pure Chemical Corporation, 5.0 mol% (1 mmol relative to 20 mmol of the substrate)) as an example of a basic compound and the hydrogen pressure was changed from 5.0 MPa to 3.0 MPa.

### (Example 67)

Dialkyl polysulfide (A) was obtained in the same manner as the manner in Example 37 except that the catalyst used in Example 65 was reused (reused eleven times) together with the zeolite, the hydrogen pressure was changed from 5.0 MPa to 3.0 MPa, and the reaction time was changed from 20 hours to 4 hours. The results are listed in Table 12.

**[Table 12]**

| Experimental number | Catalyst | Catalyst amount (mol%) | Na-X (mg) | Hydroge n pressur e (MPa) | S equ iva len t | Tern per atu re (°C ) | Tim e (h) | Con ver sio n rat io | RSH yie ld | RS₂R yie ld | RS₃R yiel d | RS₄R yie ld | RS₅R yie ld | RS₆R yie ld | RS₇R yie ld | RS₈R yie ld | RSₙR tot al yie ld |
|---|---|---|---|---|---|---|---|---|---|---|---|---|---|---|---|---|---|
| Example 56 | Co₃O₄ | 5.0 | 100 | 5.0 | 3.0 | 130 | 20 | 98 | 7 | 0 | 16 | 32 | 27 | 11 | 4 | 1 | 90 |
| Example 57 | Co₃O₄ | 5.0 | 100 | 5.0 | 3.0 | 130 | 20 | 98 | 7 | 0 | 17 | 31 | 28 | 10 | 3 | 1 | 91 |
| Example 58 | Co₃O₄ | 5.0 | 100 | 5.0 | 3.0 | 130 | 20 | 97 | 10 | 0 | 17 | 24 | 21 | 10 | 3 | 1 | 76 |
| Example 59 | Co₃O₄ | 5.0 | 100 | 7.0 | 3.0 | 130 | 20 | 97 | 10 | 0 | 19 | 27 | 23 | 8 | 2 | 1 | 81 |
| Example 60 | Co₃O₄ | 5.0 | 100 | 5.0 | 3.0 | 130 | 20 | 98 | 10 | 0 | 20 | 24 | 22 | 9 | 3 | 1 | 78 |
| Example 61 | Co₃O₄ | 5.0 | 100 (Na₂ CO₃) | 3.0 | 3.0 | 130 | 20 | 96 | 3 | 0 | 10 | 22 | 29 | 12 | 4 | 2 | 80 |
| Example 62 | Co₃O₄ | 5.0 | 100 | 5.0 | 3.0 | 130 | 20 | 97 | 10 | 0 | 19 | 26 | 25 | 10 | 3 | 1 | 84 |
| Example 63 | Co₃O₄ | 5.0 | 500 | 3.0 | 3.0 | 130 | 20 | 97 | 3 | 0 | 12 | 25 | 27 | 11 | 4 | 1 | 81 |
| Example 64 | Co₃O₄ | 5.0 | 100 | 5.0 | 3.0 | 130 | 20 | 98 | 8 | 0 | 17 | 28 | 26 | 11 | 4 | 1 | 85 |
| Example 65 | Co₃O₄ | 5.0 | 100 (die yclo hexy lami ne) | 3.0 | 3.0 | 130 | 20 | 97 | 1 | 0 | 13 | 38 | 17 | 7 | 2 | 1 | 78 |
| Example 66 | Co₃O₄ | 5.0 | 100 (NaO H) | 3.0 | 3.0 | 130 | 20 | 94 | 2 | 0 | 9 | 25 | 28 | 12 | 4 | 2 | 81 |
| Example 67 | Co₃O₄ (Reused 10 times) | 5.0 | 100 | 3.0 | 3.0 | 130 | 4 | 71 | 0 | 0 | 3 | 10 | 16 | 15 | 6 | 2 | 52 |

From the results in Table 12, it was found that in Example 59, the total yield of the dialkyl polysulfides (A) (n = 2 to 8) is 81% when the hydrogen pressure was 7.0 MPa and thus the yield of thiol was increased and the total yield of the dialkyl polysulfide (A) was decreased as compared to the total yields when the hydrogen pressure was 3.0 MPa or 5.0 MPa (Examples 30 and 37). It is presumed that the reduction reaction caused by the catalyst excessively proceeded due to hydrogen pressure exceeding a certain value.

It was found that in Example 63, when the charged amount of the zeolite was 500 mg, almost the same total yield was obtained as the total yield when the charged amount of the zeolite was 100 mg (Example 41). In other words, it was confirmed that the reaction was possible to proceed without decreasing the total yield even when the charged amount of the zeolite was five times larger.

It was found that in Examples 56 to 58, 60, 62, and 64, when the catalyst was reused 5 to 10 times together with the zeolite, the total yields were higher or nearly equal to the total yield when the catalyst was firstly used (Example 37). In particular, the ratio of dialkyl polysulfide (A) (n=3) and the yield of thiol tended to increase as the number of reuses increased. It is presumed that repeated use of the catalyst may improve the reduction ability of the catalyst.

It was found that in Example 67, in the case where the catalyst washed after 10 times reuses was further reused (reused 11 times), the conversion ratio was 71% at the stage where 4 hours had passed after the start of the reaction and thus the activity was improved.

It was found that in Examples 61, 65, and 66, when the basic compounds (Na₂Co₃, dicyclohexylamine, or NaOH) other than the Na-X type zeolite gave almost the same total yields as compared with the total yield when the Na-X type zeolite was used (Example 30).

### Industrial Applicability

The method for producing sulfurized olefin can eliminate the needs for handling hydrogen sulfide gas, which is difficult to obtain, or high pressure hydrogen sulfide gas, and the dialkyl polysulfide (A) can be obtained using hydrogen gas, which is relatively easy to obtain and handle, and thus the dialkyl polysulfide (A) can be easily produced without being affected by the supply regions of hydrogen sulfide gas. Therefore, lubricating oils or greases including the dialkyl polysulfide (A) (extreme pressure additive) and base oils can be easily produced in various regions and thus the method is extremely useful.

## Claims

1. A method for producing sulfurized olefin, the method comprising
reacting an olefin compound (a) represented by
R¹R²C=CHR³ ··· (1)
(in Formula (1), R¹ and R² each are alkyl groups; R³ is a hydrogen atom or an alkyl group, and the total number of carbon atoms of R¹, R², and R³ is 2 to 20) with sulfur in the presence of hydrogen to give dialkyl polysulfide (A).

2. The method for producing sulfurized olefin according to claim 1, wherein a pressure of supplied hydrogen in reacting the olefin compound (a) with sulfur is 0.1 MPa or more and 10 MPa or less.

3. The method for producing sulfurized olefin according to claim 1 or 2, wherein a heating temperature in reacting the olefin compound (a) with sulfur in the presence of hydrogen is 100°C or more and 200°C or less.

4. The method for producing sulfurized olefin according to claim 1 or 2, wherein the olefin compound (a) is reacted with sulfur in the presence of hydrogen and a metal element.

5. The method for producing sulfurized olefin according to claim 4, wherein the metal element is one or more metal elements selected from Group 6 to Group 11.

6. The method for producing sulfurized olefin according to claim 4, wherein the metal element is a metal element constituting a metal oxide or a metal sulfide.

7. The method for producing sulfurized olefin according to claim 4, wherein an added amount of the metal element relative to the olefin compound (a) is 0.1 mol% or more and 10 mol% or less.

8. The method for producing sulfurized olefin according to claim 4, wherein the olefin compound (a) is reacted with sulfur in the presence of hydrogen, the metal element, and zeolite.

9. The method for producing sulfurized olefin according to claim 8, wherein the zeolite has basicity.

10. The method for producing sulfurized olefin according to claim 8, wherein the zeolite has an X-type or A-type pore structure.

11. The method for producing sulfurized olefin according to claim 8, wherein an added amount of the zeolite is 2.0 parts by mass or more and 25 parts by mass or less relative to 100 parts by mass of the olefin compound (a).

12. The method for producing sulfurized olefin according to claim 1, wherein a total number of carbon atoms of R¹ and R² in the general formula (1) is 2 to 14 and R³ is a hydrogen atom.

13. The method for producing sulfurized olefin according to claim 1, wherein the olefin compound (a) is diisobutylene.
